(19) Europäisches Patentamt
European Patent Office
Office européen des brevets

(11) Veröffentlichungsnummer: **0 212 551**
**B1**

(12) # EUROPÄISCHE PATENTSCHRIFT

(45) Veröffentlichungstag der Patentschrift: **24.10.90**

(21) Anmeldenummer: **86111145.8**

(22) Anmeldetag: **12.08.86**

(51) Int. Cl.[5]: **C 07 D 471/08,**
C 07 D 471/10, A 61 K 31/495
// (C07D471/08, 241:00, 221:00),(C07D471/10, 241:00, 221:00)

(54) **Neue Tetraoxoverbindungen.**

(30) Priorität: **21.08.85 DE 3529872**

(43) Veröffentlichungstag der Anmeldung:
**04.03.87 Patentblatt 87/10**

(45) Bekanntmachung des Hinweises auf die Patenterteilung:
**24.10.90 Patentblatt 90/43**

(84) Benannte Vertragsstaaten:
**AT BE CH DE FR GB IT LI LU NL SE**

(56) Entgegenhaltungen:
**FR-A-2 375 235**

(73) Patentinhaber: **Kali-Chemie Pharma GmbH**
**Hans-Böckler-Allee 20 Postfach 220**
**D-3000 Hannover 1 (DE)**

(72) Erfinder: **Schön, Uwe, Dr. rer nat.**
**Föhrenkamp 12**
**D-3167 Burgdorf (DE)**
Erfinder: **Kehrbach, Wolfgang, Dr. rer. nat.**
**Altenbekener Damm 41**
**D-3000 Hannover 1 (DE)**
Erfinder: **Benson, Werner, Dr.**
**Boschweg 4**
**D-3016 Seelze 2 (DE)**
Erfinder: **Fuchs, Andreas, Dr.**
**Bergiusstrasse 3 B**
**D-3000 Hannover 51 (DE)**
Erfinder: **Ruhland, Michael, Dr.**
**Schwalbenflucht 24**
**D-3000 Hannover 61 (DE)**

(74) Vertreter: **Meyer, Hans J.G.**
**Kali-Chemie AG Hans-Böckler-Allee 20 Postfach 220**
**D-3000 Hannover 1 (DE)**

**EP 0 212 551 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Tetraoxoverbindungen, Verfahren zu ihrer Herstellung, Arzneimittel, enthaltend Tetraoxoverbindungen und deren pharmazeutisch verwendbare Säureadditionssalze und ein Verfahren zur Herstellung solcher Arzneimittel. Über pharmakologische Wirkungen von Verbindungen des neuen Strukturtyps ist bisher nichts bekannt.

Das Grundgerüst der erfindungsgemäßen Tetraoxoverbindungen, das 2,4,6,8-Tetraoxo-3,7-diazabicyclo-(3,3,1)-nonan sowie dessen in 3,7 und/oder 9-Stellung durch Alkyl oder Phenyl oder in 9-Stellung durch Alkylen substituierte Derivate sind bereits bekannt. Hierzu wird beispielsweise verwiesen auf Hörlein, Eur. J. Med. Chem *1977,* 301—5, Hörlein et al, Chem. Ber. *110* (1977), 3894 ff, McElvain et al, J. Amer. Chem. Soc (1958) *80,* 3915 ff, DE—OS 26 58 558, DE—OS 32 34 697.

Aufgabe der Erfindung ist es, neue Tetraoxoverbindungen mit vorteilhafter pharmakologischer Wirkung zur Verfügung zu stellen.

Aufgabe der Erfindung ist es ferner, neue Tetraoxoverbindungen zur Verfügung zu stellen, die wertvolle Zwischenprodukte zur Herstellung von pharmakologisch wirksamen neuen Tetraoxoverbindungen darstellen. Die Aufgabe wird gelöst durch die vorliegende Erfindung.

Die Erfindung betrifft neu Verbindungen der allgemeinen Formel (I)

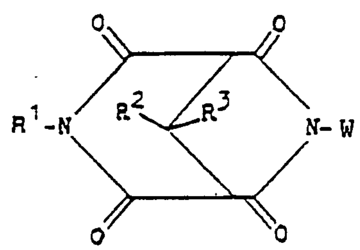

I

in der

$R^1$ eine Alkyl- oder Alkenylgruppe mit bis zu 12 Kohlenstoffatomen oder durch Phenyl oder Cycloalkyl mit 3—6 Kohlenstoffatomen substituiertes Alkyl mit 1—6 Kohlenstoffatomen bedeutet,

$R^2$ und $R^3$ unabhängig voneinander Alkyl mit 1—7 Kohlenstoffatomen oder Phenyl oder gemeinsam Alkylen mit 3—6 Kohlenstoffatomen bedeuten und

W für eine $—(CH_2)_n—Q—$Gruppe steht, worin n 2—10 bedeutet und Q

a) eine vergrängbare Fluchtgruppe X aus der Gruppe von Halogen, Tosylat-, Mesylat-, Sulfat- und Phosphatgruppen darstellt (das heißt $W = —(CH_2)_n—X$ $\triangleq$ Verbindungen (Ia) oder

b) eine $Z—R^4$-Gruppe, worin Z 1-Piperazinyl und $R^4$ in 4-Stellung stehendes Wasserstoff beduten, darstellt (das heißt $W = —(CH_2)_n—ZH$ $\triangleq$ Verbindungen Ib) oder

c) eine $Z—R^4$-Gruppe darstellt, worin Z 1-Piperazinyl bedeutet und $R^4$ eine in 4-Stellung stehende Gruppe $R^6$ ist, welche Phenyl, durch einen Substituenten aus der Gruppe Alkyl mit 1—2 Kohlenstoffatomen, Alkoxy mit 1—2 Kohlenstoffatomen, Halogen, Hydroxy, Nitro, Cyano und Trifluoromethyl monosubstituiertes oder durch Substituenten aus der Gruppe Alkyl mit 1—2 Kohlenstoffatomen, Alkoxy mit 1—2 Kohlenstoffatomen, Halogen, Trifluoromethyl, Methylendioxy und Äthylendioxy disubstituiertes Phenyl, gegebenenfalls durch Alkyl mit 1—2 Kohlenstoffatomen, Nitro, Halogen oder Alkoxy mit 1—2 Kohlenstoffatomen monosubstituiertes N-Heteroaryl aus der Gruppe 2-Pyridyl, 4-Pyridyl, 2-Pyrimidyl und 2-Pyrazinyl bedeutet (das heißt $W = —CH_2—Z—R^6$ $\triangleq$ Verbindungen Ic), sowie deren Salze und Säureadditionssalze.

Der Substituent $R_1$ enthält 1 bis 12, vorzugsweise 1 bis 7 Kohlenstoffatome.

Soweit unter $R_1$ Alkyl verstanden wird, kommt sowohl verzweigtes als auch unverzweigtes Alkyl in Frage. Unverzweigte Alkylreste sind der Methyl-, Äthyl-, n-Propyl-, n-Butyl, n-Pentyl, n-Hexyl- oder n-Heptylrest. Verzweigte Alkylreste sind der Isopropyl, sek.-Butyl-(d.h.2-Methylpropyl-), 3-Methylbutyl-, 2,2-Dimethylpropyl-, 2-Methylpentyl- und 3,3-Dimethylbutylrest.

Soweit unter $R_1$ Alkenyl verstanden wird, kommt ebenfalls sowohl verzweigtes als auch unverzweigtes, vorzugsweise unverzweigtes Alkenyl in Frage. Unverzweigte Alkenylreste sind der Allyl-(d.h.2-Propenylrest), 2-Butenyl-, 3-Butenyl-, 2-Pentenyl-, 3-Pentenyl-, 4-Pentenylrest. Verzweigte Alkenylreste sind z.B. der 2-Methyl-2-propenylrest.

In einer weiteren Variante kann $R_1$ auch einen Phenylrest oder Cyclo-Alkylrest enthalten. Dabei enthält der Cyclo-Alkylrest 3 bis 6 C-Atome. Cycloalkyl oder Phenyl-Rest sind über eine Alkenylkette mit 1 bis 6 C-Atomen, vorzugsweise 1 bis 3 C-Atomen mit den jeweiligen N-Atom verbunden. Beispiele für solche Reste sind die Cyclopropyl-, Cyclobutyl-, Cyclopentyl-, Cyclohexylgruppe, die vorzugsweise über eine Methylengruppe mit dem jeweiligen N-Atom verbunden sind.

Soweit unter $R_2$ und $R_3$ in einer bevorzugten Variante Alkyl, insbesondere unverzweigtes Alkyl verstanden wird, so gilt die für $R_1$ gemachte Angabe entsprechend. $R_2$ und $R_3$ enthalten jeweils 1 bis 7, insbesondere 1 bis 4 Kohlenstoffatome.

$R_2$ und $R_3$ können prinzipiell gleiche oder verschiedene Bedeutung besitzen. Bevorzugt wird, daß $R_2$ und $R_3$ gleiche Bedeutung besitzen.

Beispielsweise können $R_2$ und $R_3$ je Methyl und $R_1$ n-Butyl bedeuten.

Ein Sonderfall für $R_2 = R_3$ ist, daß $R_2$ und $R_3$ gemeinsam eine Alkylenkette $—(CH_2)_n—$ bilden. Dabei nimmt m Werte von 3 bis 6, insbesondere 3 bis 5 an.

Eine Untergruppe von Verbindungen gemäß Formel (I) ist dadurch gekennzeichnet, daß die Gruppe W für $(CH_2)_n—Q$ mit Q = X steht.

Diese Gruppe von Verbindungen sind wertvolle Zwischenprodukte zur Herstellung von pharmakologisch wirksamen Tetraoxoverbindungen.

Bezeichnet man zur Vereinfachung nachfolgend den Tetraoxorest

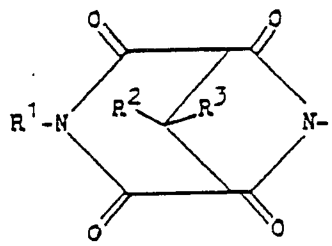

mit "T", d.h. setzt man für Formel (I) die allgemeine Form T—W, so hat die genannte Untergruppe von Verbindungen die Formel (Ia).

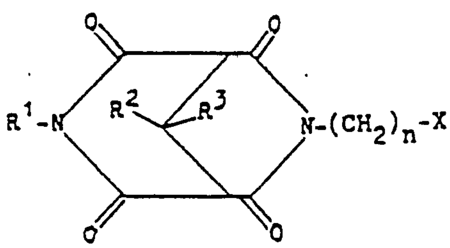

oder $T-(CH_2)_n-X$ (Ia)

Der Koeffizient n für die Alkylenkette nimmt Werte von 2 bis 10, insbesondere 2 bis 6 an.

Mit X wird eine verdrängbare Fluchtgruppe bezeichnet, insbesondere eine durch ein Amin verdrängbare Fluchtgruppe. Vorzugsweise steht X für Halogen, insbesondere Chlor oder Brom, oder eine Tosylat-, Mesylat-, Sulfat- oder Phosphat-Gruppe.

Eine weitere Untergruppe von Verbindungen sind solche, in denen W für $(CH_2)—Z—H$ steht, also Verbindungen des Typs (Ib).

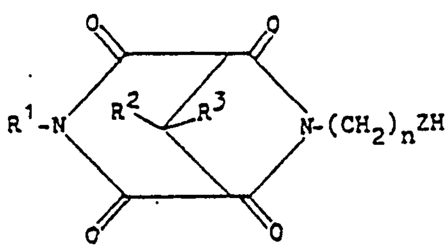

oder $T-(CH_2)_n-Z-H$ (Ib)

Hinsichtlich T und n gelten die vorstehenden Ausführungen sinngemäß; Z hat die obengenannte Bedeutung.

Auch diese Verbindungen sind wertvolle Zwischenprodukte zur Herstellung pharmakologisch wirksamer Tetraoxoverbindungen.

Eine weitere Untergruppe von Verbindungen sind jene, in denen W für $(CH_2)_n—Z—R^6$ steht, wobei $R^6$ die oben angegebene Bedeutung von $R^4$ außer Wasserstoff besitzt. Diese Verbindungen entsprechen also der allgemeinen Formel (Ic).

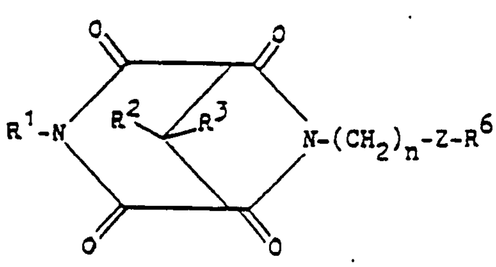

oder $T-(CH_2)_n-Z-R^6$ (Ic)

Diese Verbindungen besitzen wertvolle pharmakologische Eigenschaften.

In der Formel (Ic) haben, T, n, Z und $R^6$ die bereits angegebene Bedeutung, wobei $R^6$ Phenyl, substituiertes Phenyl, N-Heteroaryl, substituiertes N-Heteroaryl ist und wobei N-Heteroaryl ausgewählt ist aus der Gruppe 2- oder 4-Pyridyl, 2-Pyrimidyl oder 2-Pyrazinyl.

Als substituiertes Phenyl kommt für $R^6$ insbesondere die Monosubstitution in Frage. Substituenten sind dabei insbesondere Alkyl, bevorzugt 2-Alkyl wie 2-Methyl, 2-Äthyl, oder 3-Alkyl wie 3-Methyl, 3-Äthyl, oder 4-Alkyl wie 4-Methyl, 4-Äthyl oder Alkoxy, bevorzugt 2-Alkoxy wie 2-Methoxy, 2-Äthoxy oder Halogen, bevorzugt 2-Halogen wie 2-Chlor, 2-Fluor oder 3-Halogen wie 3-Chlor oder 4-Halogen wie 4-Chlor, 4-Fluor oder Hydroxy, bevorzugt 2-Hydroxy oder Nitro, bevorzugt 2-Nitro oder Cyano, bevorzugt 2-Cyano oder Trifluormethyl, bevorzugt 3-Trifluormethyl.

Als disubstituiertes Phenyl ist insbesondere zu nennen 2,6-Dialkyl, wie 2,6-Dimethy, 2,6-Diäthyl oder 3,4-Dihydroxy, 3,4-Dialkoxy, 3,4-Methylendioxy, 3,4-Äthylendioxy, 3-Trifluor-4-Halogen, insbesondere 3-Trifluor-4-Chlor.

Als substituiertes N-Heteroaryl kommt insbesondere die Monosubstitution in Frage wie für 2-Pyridyl: Alkyl, bevorzugt 5-Alkyl wie 5-Methyl, 5-Äthyl oder 4-Alkyl wie 4-Methyl, 4-Äthyl oder Nitro, bevorzugt 5-Nitro, oder Halogen, bevorzugt 5-Halogen, wie 5-Chlor oder Alkoxy, bevorzugt 6-Alkoxy, wie 6-Methoxy, 6-Äthoxy.

Besonders bevorzugte Verbindungen aus dieser Gruppe mit der allgemeinen Formel (Ic) sind jene mit folgender Kombination von Substituenten.

| $R^1$ | $R^2$ und $R^3$ | $R^4$ |
|---|---|---|
| Alkyl, ggf. substituiert durch Cycloalkyl | a) Alkyl<br>b) Alkylen<br>c) Aryl | ggf. substituiertes Phenyl |
| (Phenyl)-alkyl | a) Alkyl<br>b) Alkylen<br>c) Aryl | ggf. substituiertes Phenyl |
| Alkenyl | a) Alkyl<br>b) Alkylen<br>c) Aryl | ggf substituiertes Phenyl |
| Alkyl, ggf. substituiert durch Cycloalkyl | a) Alkyl<br>b) Alkylen<br>c) Aryl | ggf. substituiertes 2- oder 4-Pyridyl |
| (Phenyl)-alkyl | a) Alkyl<br>b) Alkylen<br>c) Aryl | ggf. substituiertes 2- oder 4-Pyridyl |
| Alkenyl | a) Alkyl<br>b) Alkylen<br>c) Aryl | ggf. substituiertes 2- oder 4-Pyridyl |
| Alkyl, ggf. substituiert durch Cycloalkyl | a) Alkyl<br>b) Alkylen<br>c) Aryl | ggf. substituiertes 2-Pyrimidyl oder 2-Pyrazinyl |
| (Phenyl)-alkyl | a) Alkyl<br>b) Alkylen<br>c) Aryl | ggf. substituiertes 2-Pyrimidyl oder 2-Pyrazinyl |
| Alkenyl | a) Alkyl<br>b) Alkylen<br>c) Aryl | ggf. substituiertes 2-Pyrimidyl oder 2-Pyrazinyl |

Die gleiche bevorzugte Substituentenkombination für $R^1$ sowie $R^2$ und $R^3$ gilt für Verbindungen der allgemeinen Formeln (Ia) und (Ib) sinngemäß.

Die Erfindung umfaßt auch Salze und Säureadditionssalze der Verbindungen (I).

Salze bedeutet dabei echte Salze z.B. von Verbindungen des Typs (Ib) mit insbesondere Natrium oder Lithium oder Salze, die mit am Aromaten stehenden, zur Salzbildung befähigten Substituenten gebildet sind, wie z.B. Phenolat-Salze.

Säureadditionssalze erhält man durch an sich bekannte Umsetzung der basischen Verbindungen (I) mit Säuren. Besonders bevorzugt sind die Säureadditionssalze von Verbindungen des Typs (Ic) mit pharmazeutisch geeigneten Säuren.

Als pharmazeutisch verwendbare Säureadditionssalze eignen sich beispielsweise wasserlösliche und wasserunlösliche Salze von anorganischen oder organischen Säuren wie z.B. das Hydrochlorid, Hydrobromid, Hydrojodid, Phosphat, Nitrat, Sulfat, Perchlorat, Acetat, Citrat, Gluconat, Benzoat, Propionat, Butyrat, Sulfosalicylat, Maleinat, Laurat, Fumarat, Succinat, Oxalat, Tartrat, Stearat, Tosylat (p-Toluolsulfonat), 2-Hydroxy-3-naphthoat, 3-Hydroxy-2-naphthoat, Mesylat (Methansulfonat), Naphthalinsulfonat.

Die Erfindung betrifft auch Arzneimittel enthaltend mindestens eine Verbindung der Formel (Ic) oder deren pharmazeutisch verwendbare Säureadditionssalze sowie ein Verfahren zur Herstellung solcher Arzneimittel, bei dem die genannten Verbindungen mit inerten, pharmazeutisch geeigneten Trägerstoffen gemischt werden und in an sich bekannter Weise in galenische Zubereitungen überführt werden. Solche Zubereitungen können z.B. sein Tabletten, Dragees, Kapseln, Pulver, Granulate, wässrige und ölige Suspensionen, Emulsionen, Sirupe oder Lösungen für die orale Anwendung, Suppositorien für die rektale Anwendung oder steril injezierbare Suspensionen oder Lösungen für die parenterale Applikation.

Die Erfindung betrifft weiterhin ein Verfahren zur Herstellung von Tetraoxoverbindungen der Formel (I), das dadurch gekennzeichnet ist, daß man eine Zwischenproduktverbindung der Formel Ia, Ib oder Id (w= H) unter geeigneten Reaktionsbedingungen mit einer Verbindung der allgemeinen Formel (II) umsetzt

$$Y—R^5 \qquad (II)$$

wobei Y und $R^5$ folgende Bedeutung haben:

a) sofern W = H ist (≙ Id), sind
a1) Y = verdrängbare Fluchtgruppe X und $R^5$ = $(CH_2)_n$—Q oder
a2) für den Fall, daß n = 4 ist,

Y- = 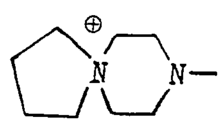 und $R^5$ = $R^6$ ,

wobei $R^6$ die Bedeutung $R^4$ außer Wasserstoff hat oder

b) sofern W = $(CH_2)_n$—X ist (≙ Ia), sind
Y = H und $R^5$ = Z—$R^4$ oder
c) sofern W = $(CH_2)_n$—ZH ist (≙ Ib), sind
Y = nucleophil vergrängbare Fluchtgruppe V, vorzugsweise Fluor, Chlor oder Brom,
$R^5$ = aktiviertes Phenyl oder ggf. substituiertes N-Heteroaryl

wobei X, Q, $R^4$, n und Z die oben angegebene Bedeutung besitzen.

Die vorstehenden allgemeinen Ausführungsformen der Verfahrensvarianten a), b) und c) stellen ein einheitliches Verfahren zur Herstellung von Verbindungen der Formel (I) dar, worin W für den Rest $(CH_2)_n$—Q, wie vorstehend beschrieben steht, das dadurch gekennzeichnet ist, daß man eine Verbindung der allgemeinen Formel T—H (Id), (Ia), oder (Ib) mit einem geeigneten Zwischenprodukt der Formel (II) in einem reaktionsinerten Lösungsmittel umsetzt.

Die als Ausgangsverbindung eingesetzten Verbindungen des Typs T—H (Id) sind zum Teil aus der bereits angeführten Literatur bekannt bzw. — sofern sie neu sind — können analog zu den bekannten Verfahren hergestellt werden. Ebenso ind die Bausteine des Typs (II) bereits bekannt, bzw. können leicht in Anlehnung an die bekannten Methoden synthetisiert werden.

Verfahren b) und c) werden herkömmlich unter Reaktionsbedingungen ausgeführt, welche man bei der Herstellung tertiärer Amine urch Alkylieren sekundärer Amine verwendet. So werden die Verbindungen der allgemeinen Formel (I) erhalten, indem man geeignete Zwischenprodukte T—H (Id), (Ia) oder (Ib) mit (II) in einem inerten Reaktionsmedium bei Temperaturen von etwa 20°C bis etwa 200°C in Gegenwart einer Base umsetzt, welche als säurebindendes Mittel brauchbar ist. Brauchbare anorganische und organische säurebindende Basen sind tert.-Amine, bevorzugt Triäthylamin, Alkali- und Erdalkalimetall-Carbonate, -Bicarbonate oder -Hydride, wobei Natriumcarbonat oder Kaliumcarbonat besonders bevorsugt sind. Die Bezeichnung "inertes Reaktionsmedium" betrifft jegliche protische oder aprotische Lösungs- oder Verdünnungsmittel, welche zu keinem wesentlichen Grad in die Reaktion eintreten. In dieser Beziehung ist

Dimethylformamid ein besonders bevorzugtes Lösungsmittel, wobei die Reaktion geeigneterweise bei Rückflußtemperatur durchgeführt wird.

In der Variante c) wird die vorgehend beschrieben eine Verbindung des Typs (Ib) mit einer Verbindung des Typs (II) umgesetzt. Als Verbindungen des Typs (II) sind bei dieser Variante substituierte aromatische Verbindungen zu verstehen, die als Teilmerkmal Y eine verdrängbare, insbesondere nucleophil verdrängbare Fluchtgruppe V aufweisen. Bevorzugte Fluchtgruppen V sind Halogen wie Fluor, Chlor, Brom. Das Teilmerkmal $R^5$ ist bei dieser Variante aktiviertes Phenyl oder substituiertes N-Heteroaryl. Aktiviertes Phenyl bedeutet Phenyl, das in ortho und/oder para-Stellung mit elektronenziehenden Gruppen substituiert ist.

Zufriedenstellende Ausbeuten an erfindungsgemäßen Verbindungen erhält man bei Reaktionszeiten von etwa 2 bis 24 Stunden. Produkte der Formel (I) können durch Kristallisationsverfahren aus Standardlösungen, wie Acetonitril, Isopropanol, Methanol, Äthanol und dergleichen oder durch jedes andere herkömmliche Verfahren wie Chromatographie unter Verwendung einer Silicagelsäule oder $Al_2O_5$ mit Mischung von Essigester, Hexan oder Alkanolen, wie Methanol und Äthanol, als Eluierungsmittel, erhalten werden.

In der Variante a) wird ein Salz, vorzugsweise ein Alkalimetallsalz des Imids der Formel (Id) alkyliert. Man verwendet herkömmliche Laboratoriumsverfahren zur Durchführung dieser Reaktion, wie sie z.B. für die Alkylierung bei der Gabriel Synthese verwendet werden. Im vorliegenden Fall werden beispielsweise die Reaktionsteilnehmer in einem inerten Reaktionsmedium bei Temperaturen von 50°C bis 200°C vereint. Toluol, Xylol und/oder Dimethylformamid sind besonders bevorzugte Lösungsmittel zur Durchführung der Reaktion, aber auch andere Lösungsmittel, welche die Reaktion oder die Reaktionsteilnehmer nicht nachteilig beeinflussen, können verwendet werden. Lösungsmittel wie Dioxan, Benzol, Aceton, Acetonitril, n-Butanol und dergleichen sind hierfür geeignet. Im allgemeinen werden die Alkalimetallsalze von Verbindungen der allgemeinen Formel (Id) hergestellt, indem man den entsprechenden Imidvorläufer mit einem Alkalihydrid, wie Natriumhydrid, einem Alkalialkoholat, wie Natriumäthoxid, einem Alkaliamid, wie Natriumamid, einer Alkalibase, wie Natriumhydroxid, Kaliumhydroxid, eine Alkalicarbonat, wie Natriumcarbonat oder Kaliumcarbonat ein einem geeigneten Lösungsmittel umsetzt. Es ist nicht notwendig, die Alkalimetallsalze der Formel (IV) vorher separat herzustellen, sie können auch in situ erzeugt werden.

In einer weiteren Ausführungsform der Variante a) werden geeignete Zwischenprodukte T—H (Id), (Ia) oder (Ib) mit (II) in einem inerten Reaktionsmedium bei Temperaturen von etwa 20°C bis etwa 200°C in Gegenwart einer Base umsetzt, welche als säurebindendes Mittel brauchbar ist. Brauchbare organische, säurebindende Basen sind tert.-Amine, bevorzugt Triäthylamin.

In der Variante a1) wird als Alkylierungsmittel die entsprechende Verbindung des Typs (II) eingesetzt. In einer Variante a2) dieses Verfahrens wird für den Fall, daß n = 4 ist, als Alkylierungsmittel das in 8-Stellung entsprechend substituierte 8-Aza-5-azoniaspiro (4,5) dekansalz eingesetzt, also eine Verbindung mit einem Kation der Struktur

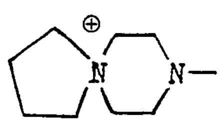

bei der als Anion ein üblicher Salzrest, insbesondere Bromid in Frage kommt.

Die Verbindungen der Formel Ic und ihre pharmakologisch akzeptablen Säureadditionssalze besitzen interessante pharmakologische Eigenschaften, insbesondere sind sie ZNS-wirksam und üben einen vorteilhaften Einfluß auf das Sozialverhalten aus. Sie zeichnen sich durch ein günstiges Wirkungsprofil mit anxiolytischen, antipsychotischen und antidepressiven Wirkungskomponenten und niedrige Toxizität aus. Die ZNS-wirksamen Eigenschaften der Substanzen können in pharmakologischen Standardtests in Tieren nachgewiesen werden.

Beschriebung der pharmakologischen Untersuchungsmethoden

In den folgenden Tabellen der Testergebnisse beziehen sich die für die Verbindungen der Formel Ic angegebenen Nummern auf die nachstehenden Herstellungsbeispiele.

1. Bestimmung der minimalen toxischen Dosis

Männlichen Mäusen von 20—25 g Gewicht werden per os und i.p. Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird als minimale toxische Dosis angegeben.

| Verbindung Nr. | Minimale toxische Dosis mg/kg Maus | |
|---|---|---|
| | p.o. | i.p. |
| 3135 | >300 | >300 |
| 3143 | >300 | >300 |
| 3107 | >300 | 200 |
| 3110 | >300 | >300 |

2. Untersuchung auf antipsychotische Eigenschaften

Zum Nachweis der antipsychotischen Eigenschaften wird die für Antipsychotica typische Hemmwirkung der Substanzen auf konditionierte Vermeidensreaktionen (= conditioned avoidance response = CAR) in Ratten untersucht.

Der CAR-Test wird in einer Versuchsanordnung nach Capaldi et al (s. R. D. Myers, Methods in Psychobiology, Academic Press, London, New York, Seiten 71—74) durchgeführt. Es werden vollautomatische Einwegkonditionierungskäfige, verwendet, welche in zwei durch einen Durchgang miteinander verbundene Kammern aufgeteilt sind und deren Boden aus einem Stahlgitter besteht, über welches in der ersten Kammer eine elektrischer Reiz verabfolgt werden kann. Die Tiere können, um diesen elektrischen Reiz zu vermeiden, aus diser ersten Kammer (Startkammer) in die zweite sichere Kammer ausweichen. An der Decke der Käfige befinden sich eine Lampe und eine Lautsprecher, welcher einen Summton abgibt.

In dem Versuchsverlauf werden die Tiere in die Startkammer gesetzt, und gleichzeitig werden Lampe und Lautsprecher als Warnsignal (= konditionierter Stimulus) eingeschaltet. Falls die Tiere bereits auf dieses Warnsignal hin in die sichere zweite Kammer überwechseln, wird dies als CAR angesehen. Falls die Tiere nicht innerhalb von 5 Sekunden in die sichere Kammer überwechseln, wird zusätzlich bis zu 15 Sekunden lang elektrischer Strom durch das Bodengitter der Startkammer gelietet (= unkonditionierter Stimulus). Sofern die Tiere auf diesen Reiz hin in die sichere Kammer überwechseln, wird dies als Fluchtreaktion (= unconditioned escape response = UER) gewertet. Sobald die Tiere in die sichere Kammer ausgewichen sind, schalten sich beide Stimuli automatisch ab.

Für den Versuch werden nur weibliche Ratten mit einem Körpergewicht von 140—210 g verwendet, welche in einem 20 Durchläufe umfassenden Training am Vortage gelernt haben, immer schon beim Einschalten von Licht und Lautsprecher zu reagieren, also eine konditionierte Vermeidensreaktion (CAR) zu zeigen. Es werden jeweils Gruppen von 4 Tieren pro Testsubstanzdosis eingesetzt, und die Tiere werden 15 mal in die Versuchssituation versetzt mit Zwischenpausen von 1,5 bis 4 Minuten. Die Mittelwerte der letzten 10 Versuche werden gewertet.

Eine Stunde vor Versuchsbeginn wird den Tieren die Testsubstanz suspendiert in einer Menge von 10 ml/kg einer 2%-igen Tyloselösung per os appliziert. Eine Kontrollgruppe erhält nur die entsprechende Menge Tyloselösung. Es wird die Dosis in µmol/mg bestimmt, welche zu einer 50%-igen Hemmung der CAR, also einer 50%-igen avoidance-Blockade führt (= $ED_{50}AB$). Fener wird die Dosis bestimmt, welche zu einer 50%-igen Hemmung der UER, also einer 50%-igen Fluchtblockade führt (= $ED_{50}EB$). Die Erbegnisse sind in der folgenden Tabelle wiedergegeben.

| Verbindung Nr. | Antipsychotische Wirkung $ED_{50}AB$ µmol/kg | $ED_{50}EB$ µmol/kg |
|---|---|---|
| 3107 | 27 | 70 |
| 3110 | 50 | 200 |
| 3150 | 57 | >215 |
| | | (215 = 43% EB) |

Die Verbindungen zeigen ein günstiges Verhältnis von AB zu EB.

3. Untersuchung der antidepressiven Eigenschaften

Die antidepressiven Eigenschaften werden durch den Nachweis der für Antidepressiva typischen

Fähigkeit der Verbindungen, die Wirkung von unterschwelligen Dosen von 5-Hydroxytryptophan (= 5-HTP) zu verstärken, gezeigt. Hierzu wird in einer Modifikation der von Corne et al beschriebenen Methode (s. Br. J. Pharmacol. *20,* 106—120) die Fähigkeit der Substanzen in mit unterschwelligen Dosen von 5-HTP behandelten Mäusen in Zusammenwirkung mit den 5-HTP ein Kopf-Schüttel-Syndrom zu erzeugen bestimmt.

Es werden männliche NMRI-Mäuse mit eine Körpergewicht von 20—30 g verwendet. 60 Minuten vor Gabe der Testsubstanzen werden den Tieren 25 mg/kg des MAO-Hemmers Pargylin, gelöst in einem Volumen von 10 ml/kg 2%-iger Tyloselösung, i.p. injiziert. Es werden Gruppen von 20 Tieren pro Dosis Testsubstanz verwendet. Die Testsubstanz wird den Tiren suspendiert in einer Menge von 10 ml/kg 2%-iger Tyloselösung per os appliziert. 30 Minuten später werden den Tieren 50 mg/kg 5-HTP gelöst in einer Menge von 10 ml/kg 2%-iger Tyloselösung i.p. appliziert und die Tiere einzeln in Käfige getan. Eine Kontrollgruppe erhält nur die Pargylin-Injektion, die 5-HTP-Injektion und p.o. Tyloselösung. 60 Minuten nach Gabe der Testsubstanzen (= 30 Minuten nach 5-HTP-Injektion) werden die Tiere 20 Sekunden lang beobachtet und festgestellt, ob das Kopf-Schüttel-Syndrom auftritt.

Als $ED_{50}$ wird diejenige Dosis in µmol/kg bestimmt, bei welcher die Anzahl der Tiere mit Kopf/Schüttel-Syndrom um 50% erhöht ist im Vergleich zu der Kontrolltiergruppe. Die Ergebnisse werden in der Nachfolgenden Tabelle wiedergegeben.

| Verbindung Nr. | Antidepressive Wirkung, in Zusammenwirkung mit 5-HTP hervorgerufenes Kopf-Schüttel-Syndrom $ED_{50}$ µ mol/kg p.o. |
|---|---|
| 3135 | 72 |
| 3142 | 74 |
| 3143 | 130 |

4. Untersuchung auf anxiolytische Eigenschaften

Zur Herstellung der anxiolytischen Wirksamkeit wird eine Modifikation einer von Davidson et al beschriebenen Methode (s. The Benzodiazepines, Raven Press New York, 1973, Seiten 327—345) verwendet. Hierbei wird untersucht, inwieweit die Substanzen fähig sind, in Ratten eine Verhaltensänderung in Konfliktsituationen zu bewirken.

Zur Herbeiführung der Konfliktsituation wird folgende Versuchanordnung verwendet. Des Versuch wird in Käfigen nach Skinner durchgeführt, welche einen elektrifizierbaren Gitterboden besitzen und mit einem von der Ratte zu bedienenden Hebel, einem automatischen Futterspender und zwei verschiedenen Lichtquellen versehen sind. Für den Test werden weibliche Wistar-Ratten mit einem Körpergewicht von 180—300 g verwendet. Zur Vorbereitung absolvieren die Tiere ein Training, in welchem sie lernen, sich Futter durch Drücken, des Hebels zu beschaffen, wobei zwei Phasen mit unterschiedlichen Bedingungen miteinander abwechseln. Die beiden Phasen sind durch verschiedene Beleuchtung voneinander unterschieden. Während der einen Phase wird eine Futterbelohnung in eine festen Verhältnis (fixed ratio) nach jedem 10. Drücken des Hebels erhalten (= FR10), jedoch wird gleichzeitig mit dem Futterpellet eine Bestrafung durch kurzzeitigen elektrischen Strom verabreicht. Während der anderen Phase wird die Futterbelohnung ohne elektrische Bestrafung erhalten, doch wird das Drücken des Hebels sehr viel weniger häufig und nur in variierenden Zeitabständen durch ein Futterpellet belohnt (Durchschnittzeitintervall 30 Sekunden = VI30). Während jeder 47 Minuten dauernden Versuchsperiode wechseln 7 VI30-Phasen (jeweils 300 Sekunden) mit 6 FR10-Phasen (jeweils 120 Sekunden).

Zur Prüfung der Testsubstanzen durchlaufen die Tiere an 4 aufeinanderfolgenden Tagen jeweils zur gleichen Zeit eine Versuchsfolge von 47 Minuten Dauer. An den Tagen 1, 2 und 3 erhalten die Tire jeweils 60 Minuten vor Versuchsbeginn per os 10 ml/kg einer 2%-igen Tyloselösung. Am Tag 4 erhalten die Tiere eine Dosis der Testsubstanz in der Tyloselösung. Für jedes Tier wird die Anzahl von Hebeldrucken in den bestraften FR10-Phasen und in den nicht bestraften VI30-Phasen separat für jeden Tag registriet. Das Mittel aus den Ergebnissen der Tage 1—3 wird jeweils als Kontrollwert für das betreffende Tier genommen. Mit diesem Kontrollwert werden die Ergebnisse des 4. Tages verglichen. Die niedrigsten Dosen, welche eine signifikante Änderung in der Anzahl von Hebeldrucken bewirken, werden sowohl für die straffreie Phase wie für die mit Strage verbundene Phase als die minimale effektive Dosis (= MED) bestimmt. Eine erhöhte Aktivität während der mit Strafe verbundenen Phase beruht auf der anxiolytischen Wirkung der Testsubstanz. Eine verringerte Aktivität während der straffreien Phase gibt einen Hinweis auf sedative Effekte der Testsubstanz. Aus den in der nachfolgenden Tabelle angegebenen Ergebnissen ist ersichtlich, daß in den Verbindungen das Verhältnis $MED_S/MED_{SF}$ vorteilhaft niedrig ist.

| Verbindung Nr. | Verhaltensänderung der Ratte in Konfliktsituation | |
|---|---|---|
| | µmol/kg p.o. | µmol/kg p.o. |
| 3107 | 14,7 | 147 |
| 3110 | 6,8 | >215 |
| 3304 | 21,5 | >215 |
| Diazepam | 31,6 | 46,4 |
| (= Vergleichssubstanz) | | |

Aufgrund Ihrer vorstehend beschreiben psycho-pharmakologischen Wirkungen eignen sich die Verbindungen der Formel Ic und ihre pharmakologisch akzeptablenen Säureadditionssalze als Psychopharmaka zur Behandlung und Prophylaxe von Erkrankungen und Funktionsstörungen des zentralen Nervensystems.

Mit den erinfundgsgemäßen Verbindungen (Ic) können Säugetiere (Menschen und Tiere) behandelt werden. Dazu verabreicht man an das zu behandelnde Säugetier systemisch eine therapeutisch wirksame Menge einer Verbindung der allgemeinen Formel (Ic) oder eines pharmazeutisch verträglichen Säureadditionssalzes davon. Eine wirksame Dosis liegt zwischen etwa 0,01 bis 40 mg/kg Körpergewicht, wobei die Dosierung davon abhängt, welche Wirkungen man erzielen möchte, welche Verabreichungsart gewählt wird und welche Verbindung man einsetzt. Ein bevorzugter Dosierungsbereich beträgt etwa 0,5 bis 1,5 mg/kg pro Tag, verabreicht in aufgeteilten Dosen. Unter systemischer Verabreichung wird eine orale, rektale und parenterale (d.h. intramuskuläire, intravenöse und subkutane) Verabreichung verstanden. Verabreicht man eine erfindungsgemäße Verbindung oral, dann ist es im allgemeinen erforderlich, eine größere Menge des Wirkstoffs einzusetzen, um denselben Effekt zu erzielen, den man in einer kleineren parenteral verabreichten Menge erzielt. Die erfindungsgemäßen Verbindungen verabreicht man vorzugsweise in solchen Konzentrationen, daß man wirksame Effekte hervorruft, ohne dabei Schädliche oder unerwünschte Nebenwirkungen hervorzurufen.

Nachfolgende Beispiele sollen die Herstellung der neuen Verbindungen näher erläutern.

Beispiel 1

Verfahrensvariante a1): Umsetzungen von Verbindungen (Id) zu Verbindungen (Ia)

Variante A)

Eine Lösung aus 0,1 mol Tetraoxoverbindung (Id), 0,1 mol 1,ω-Dibromalkan und 0,12 mol Triethylamin in 200 ml Dimethylformamid wird bei Raumtemperatur über Nacht zur Reaktion gebracht. Den während der Reaktion entstandenen Niederschlag filtriert man ab. Nach dem Abdestillieren des Lösungsmittels wird der verbleibende Rückstand mit Methylenchlorid aufgenommen und mehrmals mit Wasser gewaschen. Man trocknet die organische Phase über $MgSO_4$, filtriert das Trocknungsmittel ab und engt die Lösung ein.

Das auf diese Weise entstandene öl, kann direkt ohne weitere Reinigung weiter umgesetzt werden.

Variante B)

Eine Mischung aus 0,1 mol Tetraoxoverbindung (Id), 0,15 mol 1,ω-Dibromalkan und 0,25 mol Kaliumcarbonat in 200 ml Dimethylformamid wird über Nacht unter Rückfluß erhitzt. Nach dem Abziehen des Lösungsmittels nimmt man den verbleibenden Rückstand mit Wasser und Methylenchlorid auf. Nach dem Abtrennen der organischen Phase wäscht man die wässrige Phase noch zweimal mit Methylenchlorid nach. Die vereinigten organischen Extrakte werden über $MgSO_4$, getrocknet. Man filtriert vom Trockenmittel ab und erhält wie oben beschrieben die öligen Produkte der Formel (Ia).

Nach dieser Vorschrift sind beispielsweise die in Tabelle 1 mit Verfahrensvariante a1A) bzw. a1B) genannten Verbindungen hergestellt worden.

Beispiel 2

Verfahrensvariante a2); Umsetzung von Verbindungen (Id) zu Verbindungen (Ic)

0,1 mol Tetraoxoverbindung (Id), 0,1 mol 8-(Aryl. bzw. Heteroaryl)-8-aza-5-azoniaspiro-(4,5)-dekanbromid und 0,12 mol Natriumcarbonat werden in 500 ml Dimethylformamid für 24 Stunden unter Rückfluß erhitzt. Nach dem Abziehen des Lösungsmittels nimmt man den verbleibenden Rückstand mit Wasser auf und alkalisiert mit 10%-iger wässriger Natronlauge. Die wässrige Phase wird mehrmals mit Methylenchlorid extrahiert. Nach dem Trocknen der vereinigten organischen Extrakte über Magnesiumsulfat und Abfiltrieren des Trokenmittels destilliert man die leicht flüchtigen Anteile ab.

Falls notwendig, kann zur weiteren Reinigung eine Säulenchromatographie an Kieselgel oder Aluminiumoxid mit Essigester-Hexan-Gemischen erfolgen. Fällt die gewünschte Verbindung jedoch kristallin an, genügt zur weiteren Reinigung ein einfacher Umkristallisierungsvorgang.

Die Verbindungen der allgemeinen Formel (Id) lassen sich durch Reaktion mit einer pharmazeutisch verträglichen Säure zu einem Salz dieser Verbindung umstzen.

Nach dieser Vorschrift sind beispielsweise die in Tabelle 3 mit Verfahrensvariante a2) genannten Verbindungen hergestellt worden:

Beispiel 3

Verfahrensvariante b); Umsetzung von Verbindungen (Ia) zu Verbindungen (Ib)

Eine Mischung von 0,1 mol Verbindung (Ia), 1 mol wasserfreien Piperazin und 0,125 mol Triethylamin werden in 200 ml Dimethylformamid über Nacht bei Raumtemperatur zur Reaktion gebracht. Man engt den Ansatz im Vakuum ein und verteilt den Rückstand zwischen Wasser und Methylenchlorid. Die organische Phase wird abgetrennt. Nach dem Abfiltrieren des Trockenmittels engt man die organische Lösung im Vakuum zu einem öl ein, das man anschließend in Äthanol löst und mit äthanolischer Chlorwasserstoffsäure umsetzt. Nach dieser Vorschrift hergestellte Verbindungen sind mit Verfahrensvariante b) in Tabelle 2 angegeben.

Beispiel 4

Verfahrensvariante b); Umsetzung von Verbindungen (Ia) zu Verbindungen (Ic)

Variante A)

Eine Mischung aus 0,1 mol Verbindung (Ia), 0,1 mol Verbindung (II) und 0,125 mol Triethylamin wird in 200 ml Dimethylformamid über Nacht zur Reaktion gebracht. Falls während der Reaktion ein Niederschlag entstanden sein sollte, wird dieser abfiltriert. Die klare Lösung wird bis zur Trocknene eingeengt. Den verbleibenden Rückstand verteilt man zwischen Wasser und Methylenchlorid. Die organische Phase wird abgetrennt, zweimal mit Wasser gewaschen und über $MgSO_4$ getrocknet. Nach dem Abfiltrieren des Trockenmittels engt man die organische Lösung ein.

Falls notwendig, kann zur weiteren Reinigung eine Säulenchromatographie an Kieselgel oder Aluminiumoxid mit Essigester-Hexan-Gemischen erfolgen. Fällt die gewünschte Verbindung jedoch kristallin an genügt zur weiteren Reinigung ein einfacher Umkristallisationsvorgang.

Variante B)

Eine Mischung aus 0,1 mol Verbindung (Ia), 0,1 mol Verbindung II und 0.25 mol Kaliumcarbonat in 250 ml Dimethylformamid wird über Nächt unter Rückfluß erhitzt. Nach dem Abziehen des Lösungsmittels verteilt man den Rückstand zwischen Wasser und Methylenchlorid und arbeitet wie unter Variante A beschrieben weiter auf.

Nach diesen beiden Vorschriften sind beispielsweise die in Tabelle 3 unter Verfahrensvariante bA) bzw. bB) genannten Verbindungen herstellt worden:

Beispiel 5

Verfahrensvariante a1), Umsetzung von Verbindungen Id) zu Verbindungen (Ic)

Eine Mischung von 0,1 mol Verbindung (Id), 0,1 mol Verbindung (II) und 0,12 mol Natriumcarbonat wird in 300 ml Dimethylformamid für 7 Stunden am Rückfluß erhitzt. Nach dem Abziehen des Lösungsmittels nimmt man den verbleibenden Rückstand mit Wasser auf und alkalisiert mit 10% iger wässriger Natronlauge. Die wässrige Phase wird mehrmals mit Methylenchlorid extrahiert. Nach dem Trocknen der vereinigten organischen Extrakte über $MgSO_4$, destilliert man die leicht flüchtigen Anteile ab.

Das zurückbleibende öl wird in Isopropanol gelöst und mit in Isopropanol gelöster Chlorwasserstoffsäure versetzt.

Nach dieser Vorschrift sind beispielsweise die in Tabelle 3 mit Verfahrensvariante a1C) genannten Verbindungen hergestellt worden.

Beispiel 6

Verfahrensvariante c). Umsetzung von Verbindungen (Ib) zu Verbindungen (Ic)

Eine Mischung aus 0,05 mol Verbindung Nr. 2101 (Base) als beispielhafte Verbindung des Typs (Ib), 0,05 mol 2-Chlorpyrimidin als beispielhafte Verbindung des Typs (II) und 0,1 mol Kaliumcarbonat wird über Nacht in 200 ml Acetonitril am Rückfluß erhitzt. Nach dem Abziehen des Lösungsmittels verteilt man den verbleibenden Rückstand zwischen Wasser und Methylenchlorid. Die organische Phase wird abgetrennt und über $MgSO_4$ getrocknet. Nach dem Abfiltrieren vom Trockenmittel engt man die organische Lösung in Vakuum ein. Das zurückbleibende öl wird in Ethanol gelöst und mit ethanolischer Chlorwasserstoffsäure versetzt. Das isolierte Salz (Verbindung 3304) enthält 2,1 Equivalente HCl und schmilzt zwischen 216 und 218°C. Analog zu dieser Vorschrift wurden die in Tabelle 3 unter Verfahrensvariante c) genannten Verbindungen hergestellte.

Beispiel 7 — Tabletten

Zusammensetzung:

| | |
|---|---|
| Wirkstoff Nr. 3209 | 20 Teile |
| Maisstärke | 30 Teile |
| Lactose | 55 Teile |
| Kollidon 25® | 5 Teile |
| Magnesiumstearat | 2 Teile |
| Hydriertes Rizinusöl | 1 Teil |
| Total | 113 Teile |

Herstellungsvorschrift:

Der Wirkstoff wird mit Maisstärke und feinpulvriger Lactose in einem Mischer vermischt. Die entstandene Mischung wird mit einer 20 %igen Lösung von Polyvinylpyrrolidon, (Kollidon 25, Fa. BASF) in Isopropanol durchfeuchtet. Falls erforderlich, wird weiteres Isopropanol hinzugefüht. Das feuchte Granulat wird durch ein 2 mm-Sieb passiert, bei 40°C auf Horden getrocknet und anschließend durch ein 1 mm-Sieb (Frewitt-Maschine) passiert. Nach dem Vermischen des Granulats mit Magnesiumstearat und hydriertem Rizinusöl werden damit Tabletten von 113 mg gepreßt, so daß jede Tablette 20 mg Wirkstoff enthält.

Beispiel 8 — Kapseln

Zusammensetzung:

| | |
|---|---|
| Wirkstoff Nr. 3209 | 20 Teile |
| Maistärke | 20 Teile |
| Lactose | 45 Teile |
| Kollidon 25® | 3 Teile |
| Magnesiumstearat | 1,5 Teile |
| Aerosil 200® | 0,5 Teile |
| Total | 90 Teile |

Herstellungsvorschrift:

Der Wirkstoff wird mit Maisstärke und feinpulvriger Lactose in einem Mischer vermischt. Die entstandene Mischung wird mit einer 20 %igen Lösung von Polyvinylpyrrolidon, (Kollidon 25) in Isopropanol durchgefeuchtet. Falls erforderlich, wird weiteres Isopropanol hinzugefüht. Das feuchte Granulat wird durch ein 1,6 mm-Sieb (Frewitt) passiert, bis 40°C auf Horden getrocknet und anschließend durch ein 1 mm-Sieb (Frewitt) passiert. Nach dem Vermischen des Granulats mit Magnesiumstearat und Kieselsäureaerogel (Aerosil 200, Fa. Degussa) füllt man davon jeweils 90 mg mittels automatischer Kapselmaschine in Hargelatinekapseln der Größe 4 ab, so daß jede Kapsel 20 mg Wirkstoff anthält.

Beispiel 9 — Ampullen

Zusammensetzung (pro Ampulle):

| | |
|---|---|
| Wirkstoff Nr. 3209 | 5 mg |
| Natriumchlorid | 16 mg |
| Wasser fur Injektionszwecke | ad 2,0 ml |

Herstellungsvorschrift:

Man löst Natriumchlorid in Wasser für Injektionszwecke auf, fügt den Wirkstoff hinzu und löst unter

Rühren. Mit genügend Wasser für Injektionszwecke wird bis zum Endvolumen aufgefüllt. Der Ansatz wird durch ein Membranfilter 0,25 µ passiert. Man füllt in Braunglasampullen jeweils 2,15 ml ab und schmilzt sie zu. Bei 121°C wird 30 Minuten lang mit Dampf sterilisiert. 2 ml Injektionslösung enthalten 5 mg Wirkstoff.

In den folgenden Tabellen werden außer den bereits genannten Bezeichnungen folgende Abkürzungen verwendet: B = Base, S = Salz, Ph = Phenyl, Py = Pyridyl, Pm = Pyrimidyl, Pz = Pyrazinyl, ch = Cyclohexyl. So wird beispielsweise ein Substituent 2-Methoxyphenyl als Ph-2-$OCH_3$ und 4-Methyl-2-pyridyl als 2-Py-4-$CH_3$, bezeichnet.

TABELLE 1: Verbindungen des Typs (Ia)

| Nr. | $R^1$ | $R^2$ | $R^3$ | n | X | Schmelzpunkt (°C) | Variante |
|---|---|---|---|---|---|---|---|
| 1101 | $C_2H_5$ | $CH_3$ | $CH_3$ | 4 | Br | 125—127 | a1A |
| 1102 | n-$C_4H_9$ | $CH_3$ | $CH_3$ | 2 | Br | 71—74 | a1B |
| 1103 | n-$C_4H_9$ | $CH_3$ | $CH_3$ | 3 | Br | 77—79 | a1A |
| 1104 | n-$C_4H_9$ | $CH_3$ | $CH_3$ | 4 | Br | öl | a1A |
| 1105 | n-$C_4H_9$ | $CH_3$ | $CH_3$ | 5 | Br | 65—69 | a1A |
| 1106 | n-$C_6H_{13}$ | $CH_3$ | $CH_3$ | 4 | Br | 45—48 | a1A |
| 1107 | n-$C_6H_{13}$ | $CH_3$ | $CH_3$ | 5 | Br | 52—55 | a1A |

TABELLE 2: Verbindungen des Typs (Ib)

| Nr. | $R^1$ | $R_2$ | $R_3$ | n | Schmelzpunkt (°C) Base/Salz | Variante |
|---|---|---|---|---|---|---|
| 2101 | n-$C_4H_9$ | $CH_3$ | $CH_3$ | 4 | S (2 HCL) 202—205 | b |

TABELLE 3: Verbindungen des Typs (Ic)

| Nr. | $R^1$ | $R^2$ | $R^3$ | $R^4$ | n | Schmelzpunkt (°C) Base/Salz | Verfahren |
|---|---|---|---|---|---|---|---|
| 3101 | $C_2H_5$ | $CH_3$ | $CH_3$ | Ph-2-$OCH_3$ | 4 | B: 125—129 | bA |
| 3102 | n-$C_4H_9$ | $CH_3$ | $CH_3$ | Ph | 3 | B: 106 | bA |
| 3103 | n-$C_4H_9$ | $CH_3$ | $CH_3$ | Ph | 4 | B: 110—112 | bB |
| 3104 | n-$C_4H_9$ | $CH_3$ | $CH_3$ | Ph | 5 | B: 80—82 | bA |
| 3105 | n-$C_4H_9$ | $CH_3$ | $CH_3$ | Ph-2-$OCH_3$ | 2 | B: 125—128 | bB |
| 3106 | n-$C_4H_9$ | $CH_3$ | $CH_3$ | Ph-2-$OCH_3$ | 3 | B: 100 | bA |
| 3107 | n-$C_4H_9$ | $CH_3$ | $CH_3$ | Ph-2-$OCH_3$ | 4 | B: 103—105 | bB |
| 3108 | n-$C_4H_9$ | $CH_3$ | $CH_3$ | Ph-2-$OCH_3$ | 5 | B: 80—82 | bB |
| 3109 | n-$C_4H_9$ | $CH_3$ | $CH_3$ | Ph-2-$OCH_3$ | 6 | B: 62—67 | bA |
| 3110 | n-$C_4H_9$ | $CH_3$ | $CH_3$ | Ph-2-$OC_2H_5$ | 4 | B: 100—102 | bA |
| 3111 | n-$C_4H_9$ | $CH_3$ | $CH_3$ | Ph-2-$OC_2H_5$ | 5 | B: 236—240 | bA |
| 3112 | n-$C_4H_9$ | $CH_3$ | $CH_3$ | Ph-2-$CH_3$ | 3 | B: 220—230 | bB |

TABELLE 3: Verbindungen des Typs (Ic) (Fortsetzung)

| Nr. | $R^1$ | $R^2$ | $R^3$ | $R^4$ | n | Schmelzpunkt (°C) Base/Salz | Verfahren |
|---|---|---|---|---|---|---|---|
| 3113 | n-$C_4H_9$ | $CH_3$ | $CH_3$ | Ph-2-$CH_3$ | 4 | B: 103—105 | a2 |
| 3150 | sec $C_4H_9$ | $CH_3$ | $CH_3$ | Ph-2-$OCH_3$ | 4 | B: 130—132 | a2 |
| 3151 | $CH_2$-Ch | $CH_3$ | $CH_3$ | Ph-2-$OCH_3$ | 4 | S: (1WS) 86—90 | a2 |
| 3176 | $CH_2$-Ph | $CH_3$ | $CH_3$ | PH-2-$OCH_3$ | 4 | B: 129—131 | a2 |
| 3181 | $CH_2$-Ph | —$(CH_2)_5$— | | Ph-2-$OCH_4$ | 4 | S: (1WS) 103—106 | a2 |
| 3114 | n-$C_4H_9$ | $CH_3$ | $CH_3$ | Ph-2-Cl | 3 | B: 88—90 | bA |
| 3115 | n-$C_4H_9$ | $CH_3$ | $CH_3$ | Ph-2-Cl | 4 | B: 122—124 | a2 |
| 3116 | n-$C_4H_9$ | $CH_3$ | $CH_3$ | Ph-2-$NO_2$ | 4 | öl | bA |
| 3117 | n-$C_4H_9$ | $CH_3$ | $CH_3$ | Ph-2-$NO_2$ | 5 | B: 90—92 | bA |
| 3118 | n-$C_4H_9$ | $CH_3$ | $CH_3$ | Ph-2-CN | 4 | B: 158—160 | bA |
| 3119 | n-$C_4H_9$ | $CH_3$ | $CH_3$ | Ph-2-CN | 5 | B: 233—235 | bA |
| 3120 | n-$C_4H_9$ | $CH_3$ | $CH_3$ | Ph-2-OH | 4 | S (HBr) 236—238 | bA |
| 3121 | n-$C_4H_9$ | $CH_3$ | $CH_3$ | Ph-2-OH | 5 | S (HBr) 175—180 | bA |
| 3122 | n-$C_4H_9$ | $CH_3$ | $CH_3$ | Ph-2,6-$(CH_3)_2$ | 4 | B: 123—127 | bA |
| 3123 | n-$C_4H_9$ | $CH_3$ | $CH_3$ | Ph-3-$CH_3$ | 3 | B: 202—205 | bA |
| 3124 | n-$C_4H_9$ | $CH_3$ | $CH_3$ | Ph-3-$CH_3$ | 4 | B: 115 | a2 |
| 3126 | n-$C_4H_9$ | $CH_3$ | $CH_3$ | Ph-3-$CH_3$ | 5 | öl | bA |
| 3127 | n-$C_4H_9$ | $CH_3$ | $CH_3$ | Ph-3-$OCH_3$ | 4 | öl | a2 |
| 3128 | n-$C_4H_9$ | $CH_3$ | $CH_3$ | Ph-3-Cl | 3 | B: 89—92 | bB |
| 3129 | n-$C_4H_9$ | $CH_3$ | $CH_3$ | Ph-3-Cl | 4 | B: 120 | a2 |
| 3130 | n-$C_4H_9$ | $CH_3$ | $CH_3$ | Ph-3-$CF_3$ | 4 | öl | bA |
| 3131 | n-$C_4H_9$ | $CH_3$ | $CH_3$ | Ph-4-$CH_3$ | 3 | B: 116—118 | bA |
| 3132 | n-$C_4H_9$ | $CH_3$ | $CH_3$ | Ph-4-$CH_3$ | 4 | B: 125—128 | a2 |
| 3133 | n-$C_4H_9$ | $CH_3$ | $CH_3$ | Ph-4-Cl | 3 | B: 255—260 | bB |
| 3134 | n-$C_4H_9$ | $CH_3$ | $CH_3$ | Ph-4-Cl | 4 | B: 134 | a2 |
| 3135 | n-$C_4H_9$ | $CH_3$ | $CH_3$ | Ph-4-F | 4 | öl | bB |
| 3136 | n-$C_4H_9$ | $CH_3$ | $CH_3$ | Ph-4-$OCH_3$ | 4 | B: 128—130 | bB |
| 3137 | n-$C_4H_9$ | $CH_3$ | $CH_3$ | Ph-3-$CF_3$-4-Cl | 4 | öl | bB |
| 3138 | n-$C_4H_9$ | $CH_3$ | $CH_3$ | Ph-3,4-(OCH2O) | 4 | B: 125—127 | bB |
| 3139 | n-$C_6H_{13}$ | $CH_3$ | $CH_3$ | Ph | 4 | B: 57 | bA |
| 3140 | n-$C_6H_{13}$ | $CH_3$ | $CH_3$ | Ph | 5 | B: 68—70 | bA |

TABELLE 3: Verbindungen des Typs (Ic) (Fortsetzung)

| Nr. | $R^1$ | $R^2$ | $R^3$ | $R^4$ | n | Schmelzpunkt (°C) Base/Salz | Verfahren |
|---|---|---|---|---|---|---|---|
| 3141 | n-$C_6H_{13}$ | $CH_3$ | $CH_3$ | Ph-2-$OCH_3$ | 4 | B: 103—105 | bA |
| 3142 | n-$C_6H_{13}$ | $CH_3$ | $CH_3$ | Ph-2-$OCH_3$ | 5 | B: 74—76 | bA |
| 3146 | n-$C_6H_{13}$ | $CH_3$ | $CH_3$ | Ph-3-$CH_3$ | 4 | B: 103—105 | bA |
| 3143 | n-$C_6H_{13}$ | $CH_3$ | $CH_3$ | Ph-3-$CH_3$ | 5 | B: 86—89 | bA |
| 3144 | n-$C_6H_{13}$ | $CH_3$ | $CH_3$ | Ph-4-F | 4 | B: 66—68 | bA |
| 3145 | N-$C_6H_{13}$ | $CH_3$ | $CH_3$ | Ph-4-F | 5 | B: öl | bA |
| 3201 | $C_2H_5$ | $CH_3$ | $CH_3$ | 2-Py | 4 | B: 124—125 | bA |
| 3202 | n-$C_4H_9$ | $CH_3$ | $CH_3$ | 2-Py | 2 | B: 111—114 | bB |
| 3203 | n-$C_4H_9$ | $CH_3$ | $CH_3$ | 2-Py | 3 | S (3 WS) 78—84 | bB |
| 3204 | n-$C_4H_9$ | $CH_3$ | $CH_3$ | 2-Py | 4 | S (3 WS) 87—90 | a2 |
| 3205 | n-$C_4H_9$ | $CH_3$ | $CH_3$ | 2-Py | 5 | S (3,1 WS) 74—77 | bB |
| 3206 | n-$C_4H_9$ | $CH_3$ | $CH_3$ | 2-Py | 6 | B: 106—109 | bA |
| 3207 | n-$C_4H_9$ | $CH_3$ | $CH_3$ | 4-Py | 4 | S (2,9 WS) 93—97 | bA |
| 3208 | n-$C_4H_9$ | $CH_3$ | $CH_3$ | 2-Py-4-$CH_3$ | 3 | S (3 WS) 89—95 | bB |
| 3209 | n-$C_4H_9$ | $CH_3$ | $CH_3$ | 2-Py-4-$CH_3$ | 4 | S (3 WS) 105—110 | bB |
| 3210 | n-$C_4H_9$ | $CH_3$ | $CH_3$ | 2-Py-5-$CH_3$ | 4 | B: 110—112 | bA |
| 3211 | n-$C_4H_9$ | $CH_3$ | $CH_3$ | 2-Py-5-Cl | 4 | B: 113—115 | bA |
| 3212 | n-$C_4H_9$ | $CH_3$ | $CH_3$ | 2-Py-5-$NO_2$ | 4 | B: 97—99 | bA |
| 3213 | n-$C_4H_9$ | $CH_3$ | $CH_3$ | 2-Py-6-$OCH_3$ | 4 | B: 113—116 | bA |
| 3261 | n-$C_4H_9$ | $-(CH_2)_4-$ | | 2-Py | 4 | S (3 WS) 128—132 | bB |
| 3262 | n-$C_4H_9$ | $-(CH_2)_5-$ | | 2-Py | 4 | S (WS) 155—165 | a2 |
| 3301 | $C_2H_5$ | $CH_3$ | $CH_3$ | 2-Pm | 4 | B: 142—144 | bA |
| 3302 | n-$C_4H_9$ | $CH_3$ | $CH_3$ | 2-Pm | 2 | S: (1,8 HCl) 210—212 | a1C, bA |
| 3303 | n-$C_4H_9$ | $CH_3$ | $CH_3$ | 2-Pm | 3 | B: 107—108 | bA |
| 3304 | n-$C_4H_9$ | $CH_3$ | $CH_3$ | 2-Pm | 4 | S (2,1 HCl) 216—218 | c; bB |
| 3305 | n-$C_4H_9$ | $CH_3$ | $CH_3$ | 2-Pm | 5 | S (1,4 HCl) 184—186 | bA |
| 3306 | n-$C_4H_9$ | $CH_3$ | $CH_3$ | 2-Pm | 6 | S (1,7 HCl) 129 | bA |
| 3307 | n-$C_4H_9$ | $CH_3$ | $CH_3$ | 2-Pz | 4 | B: 103—105 | bA |
| 3361 | n-$C_4H_9$ | $-(CH_2)_4-$ | | 2-Pm | 4 | S (2,5 HCl) 205—207 | bB |
| 3362 | n-$C_4H_9$ | $-(CH_2)_5-$ | | 2-Pm | 4 | S (2 HCl) 215—217 | bB |

**Patentansprüche für die Vertragsstaaten: BE CH DE FR GB IT LU NL SE**

1. Tetraoxoverbindungen der Formel (Ic)

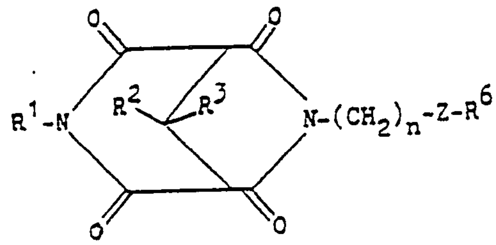

Ic

in der

$R^1$ eine Alkyl- oder Alkenylgruppe mit bis zu 12 Kohlenstoffatomen oder durch Phenyl oder Cycloalkyl mit 3—6 Kohlenstoffatomen substituiertes Alkyl mit 1—6 Kohlenstoffatomen bedeutet,

$R^2$ und $R^3$ unabhängig voneinander Alkyl mit 1—7 Kohlenstoffatomen oder Phenyl oder gemeinsam Alkylen mit 3—6 Kohlenstoffatomen bedeuten,

n 2—10 Kohlenstoffatome bedeutet,

Z die 1-Piperazinylgruppe bedeutet, und

$R^6$ in 4-Stellung stehendes Phenyl, durch einen Substituenten aus der Gruppe Alkyl mit 1—2 Kohlenstoffatomen, Alkoxy mit 1—2 Kohlenstoffatomen, Halogen, Hydroxy, Nitro, Cyano und Trifluormethyl monosubstituiertes oder durch Substituenten aus der Gruppe Alkyl mit 1—2 Kohlenstoffatomen, Alkoxy mit 1—2 Kohlenstoffatomen, Halogen, Trifluormethyl, Methylendioxy und Äthylendioxy disubstituiertes Phenyl, gegebenenfalls durch Alkyl mit 1—2 Kohlenstoffatomen, Nitro, Halogen oder Alkoxy mit 1—2 Kohlenstoffatomen monosubstituiertes N-Heteroaryl aus der Gruppe 2-Pyridyl, 4-Pyridyl, 2-Pyrimidyl und 2-Pyrazinyl bedeutet,

sowie deren Salze und Säureadditionssalze.

2. Verbindungen gemäß Anspruch 1, in denen $R^1$ 1 bis 7 C-Atome enthält.

3. Verbindungen gemäß Anspruch 1 oder 2, in denen $R^1$ unverzweigtes Alkyl oder Alkenyl bedeutet.

4. Verbindungen gemäß Anspruch 1 oder 2, in denen $R^1$ verzweigtes Alkyl oder Alkenyl bedeutet.

5. Verbindungen gemäß Anspruch 1 oder 2, in denen $R^1$ einen über einen Alkylenrest mit 1 bis 6 C-Atomen mit dem N-Atom verknüpften Phenyl- oder Cycloalkylrest bedeutet.

6. Verbindungen gemäß einem der vorhergehenden Ansprüche, in denen $R^2$ und/oder $R^3$ Alkyl mit 1 bis 4 C-Atomen bedeuten.

7. Verbindungen gemäß Anspruch 6, in denen $R^2$ und/oder $R^3$ unverzweigtes Alkyl bedeuten.

8. Verbindungen gemäß Anspruch 1 bis 5, in denen $R^2$ und $R^3$ gemeinsam eine Alkylenkette $-(CH_2)_m-$ mit m = 3 bis 6 bilden.

9. Verbindungen gemäß einem der vorhergehenden Ansprüche, in denen $R^2$ und $R^3$ gleich sind.

10. Verbindungen gemäß einem der vorhergehenden Ansprüche in denen die Alkylenkette $-(CH_2)_n-$ 2 bis 6 C-Atome enthält.

11. Verbindungen gemäß Anspruch 1, in denen $R^6$ monosubstituiertes Phenyl bedeutet.

12. Verbindungen gemäß Anspruch 11, worin $R^6$ 2-Methoxy- oder 4-Chlorphenyl bedeutet.

13. Verbindungen gemäß Anspruch 1, 11 und 12, in denen $R^1$ n-Butyl und $R^2$ und $R^3$ Metyl bedeuten.

14. Tetraoxoverbindungen der Formel Ia und Ib

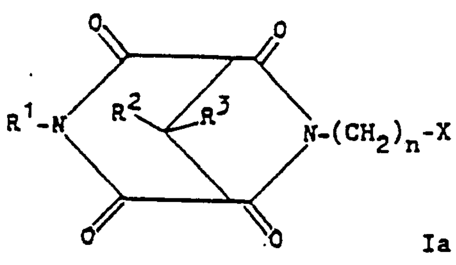

Ia

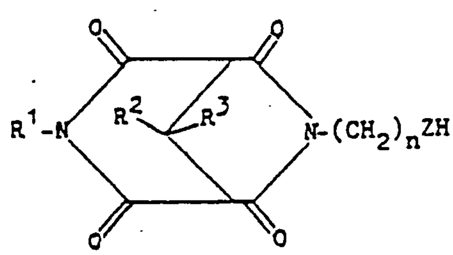

Ib

worin

$R^1$ eine Alkyl- oder Alkenylgruppe mit bis zu 12 Kohlenstoffatomen oder durch Phenyl oder Cycloalkyl mit 3—6 Kohlenstoffatomen substituiertes Alkyl mit 1—6 Kohlenstoffatomen bedeutet,

$R^2$ und $R^3$ unabhängig voneinander Alkyl mit 1—7 Kohlenstoffatomen oder Phenyl oder gemeinsam Alkylen mit 3—6 Kohlenstoffatomen bedeuten,

n 2—10 bedeutet,

X eine verdrängbare Fluchtgruppe aus der Gruppe von Halogen, Tosylat-, Mesylat-, Sulfat- und Phosphatgruppen bedeutet, und

Z die 1-Piperazinylgruppe bedeutet.

15. Arzneimittel enthaltend mindestens eine Verbindung gemäß Anspruch 1 oder deren pharmazeutisch verwendbare Säureadditionssalze.

16. Verfahren zur Herstellung von Verbindungen der Formel Ic gemäß Anspruch 1

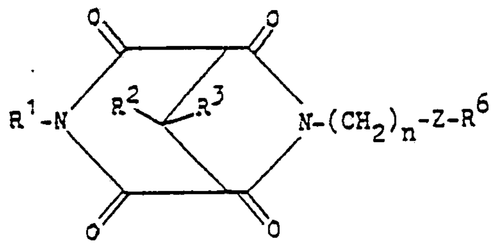

Ic

worin $R^1$, $R^2$, $R^3$, $R^6$, n und Z die in Anspruch 1 angegebene Bedeutung besitzen, und deren Salzen und Säureadditionssalzen, dadurch gekennzeichnet, daß man Verbindungen der Formel Ia oder Ib oder Id

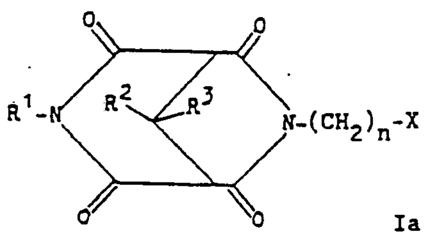

Ia

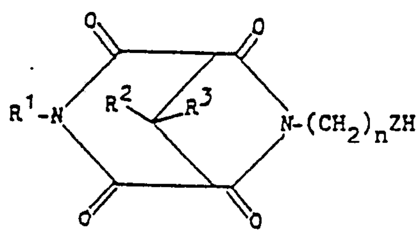

Ib

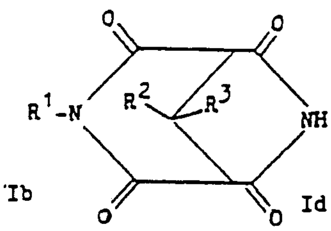

Id

worin $R^1$, $R^2$, $R^3$, n und Z obige Bedeutung besitzen, und X die in Anspruch 14 angegebene Bedeutung besitzt, mit einer Verbindung der allgemeinen Formel (II) umsetzt,

$$Y—R^5 \qquad (II)$$

wobei

a) für den Fall der Umsetzung mit einer Verbindung der Formel Id,

a1) Y die in Anspruch 14 definierte verdrängbare Fluchtgruppe X und $R^5$ eine $(CH_2)_n—Z—R^6$-Gruppe, worin n, Z und $R^6$ die in Anspruch 1 angegebene Bedeutung besitzen, darstellen oder

a2) für den Fall, daß n = 4 ist, Y die Gruppe

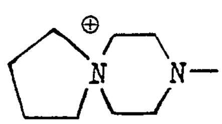

und $R^5$ den Rest $R^6$ darstellen, oder

b) für den Fall der Umsetzung mit einer Verbindung der Formel Ia

Y Wasserstoff und $R^5$ eine $Z—R^6$-Gruppe, worin Z und $R^6$ die in Anspruch 1 angegebene Bedeutung besitzen, darstellen oder

c) für den Fall der Umsetzung mit einer Verbindung der Formel Ib

Y eine nukleophil verdrängbare Fluchtgruppe V und $R^5$ ein in ortho- und/oder para-Stellung mit elektronenziehenden Substituenten aus der Gruppe der in Anspruch 1 angegebenen Substituenten substituiertes Phenyl oder ein substituiertes N-Heteroaryl gemäß Anspruch 1 darstellen,

und die erhalten Verbindungen der Formel I gewünschtenfalls in ihre Salze oder Säureadditionssalze überführt.

17. Verfahren zur Herstellung von Verbindungen der Formeln Ia und Ib gemäß Anspruch 14

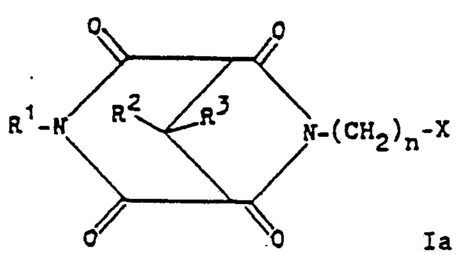

Ia

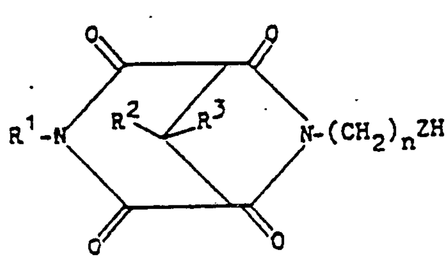

Ib

worin $R^1$, $R^2$, $R^3$, n, Z und X die in Anspruch 14 angegebene Bedeutung besitzen, dadurch gekennzeichnet, daß man

a) eine Verbindung der Formel Id

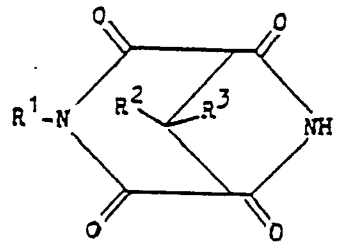

Id

worin $R^1$, $R^2$ und $R^3$ die in Anspruch 14 angegebene Bedeutung besitzen,

mit einer Verbindung der Formel (II) umsetzt

$$Y—R^5 \qquad (II)$$

**Patentansprüche für den Vertragsstaat: AT**

1. Verfahren zur Herstellung von Tetraoxoverbindungen der Formel (Ic)

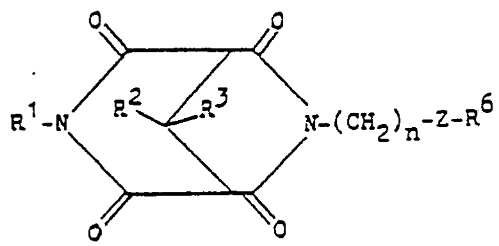

Ic

in der

$R^1$ eine Alkyl- oder Alkenylgruppe mit bis zu 12 Kohlenstoffatomen oder durch Phenyl oder Cycloalkyl mit 3—6 Kohlenstoffatomen substituiertes Alkyl mit 1—6 Kohlenstoffatomen bedeutet,

$R^2$ und $R^3$ unabhängig voneinander Alkyl mit 1—7 Kohlenstoffatomen oder Phenyl oder gemeinsam Alkylen mit 3—6 Kohlenstoffatomen bedeuten,

n 2—10 Kohlenstoffatome bedeutet,

Z die 1-Piperazinylgruppe bedeutet, und

$R^6$ in 4-Stellung stehendes Phenyl, durch einen Substituenten aus der Gruppe Alkyl mit 1—2 Kohlenstoffatomen, Alkoxy mit 1—2 Kohlenstoffatomen, Halogen, Hydroxy, Nitro, Cyano und Trifluormethyl monosubstituiertes oder durch Substituenten aus der Gruppe Alkyl mit 1—2 Kohlenstoffatomen, Alkoxy mit 1—2 Kohlenstoffatomen, Halogen, Trifluormethyl, Methylendioxy und Äthylendioxy disubstituiertes Phenyl, gegebenenfalls durch Alkyl mit 1—2 Kohlenstoffatomen, Nitro, Halogen oder Alkoxy mit 1—2 Kohlenstoffatomen monosubstituiertes N-Heteroaryl aus der Gruppe 2-Pyridyl, 4-Pyridyl, 2-Pyrimidyl und 2-Pyrazinyl bedeutet,

sowie deren Salze und Säureadditionssalze,

sowie deren Salzen und Säureadditionssalzen, dadurch gekennzeichnet, daß man Verbindungen der Formel Ia oder Ib oder Id

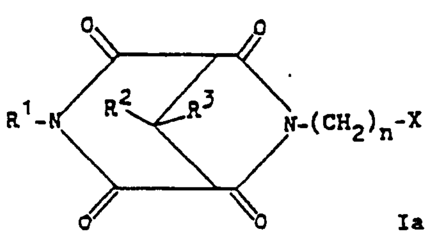

Ia

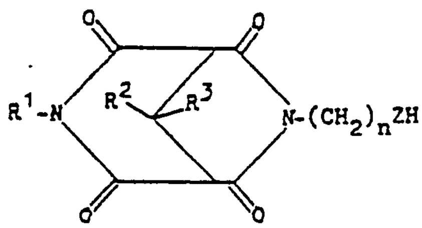

Ib

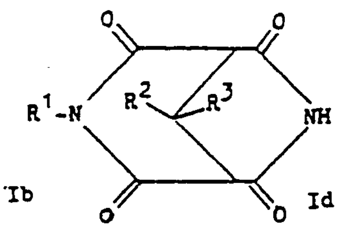

Id

worin $R^1$, $R^2$, $R^3$, n und Z obige Bedeutung besitzen, und X eine verdrängbare Fluchtgruppe aus der Gruppe von Halogen, Tosylat-, Mesylat-, Sulfat- under Phosphatgruppen, bedeutet, mit einer Verbindung der allgemeinen Formel (II) umsetzt,

$$Y—R^5 \qquad (II)$$

wobei

a) für den Fall der Umsetzung mit einer Verbindung der Formel Id,

a1) Y die obenstehend definierte verdrängbare Fluchtgruppe X und $R^5$ eine $(CH_2)_n$—Z—$R^6$-Gruppe, worin n, Z und $R^6$ die oben angegebene Bedeutung besitzen, darstellen oder

a2) für den Fall, daß n = 4 ist, Y die Gruppe

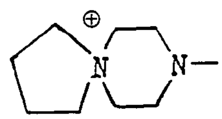

und $R^5$ den Rest $R^6$ darstellen, oder

b) für den Fall der Umsetzung mit einer Verbindung der Formel Ia

Y Wasserstoff und $R^5$ eine Z—$R^6$-Gruppe, worin Z und $R^6$ die in oben angegebene Bedeutung besitzen, darstellen oder

c) für den Fall der Umsetzung mit einer Verbindung der Formel Ib

Y eine nukleophil verdrängbare Fluchtgruppe V und $R^5$ ein in ortho- und/oder para-Stellung mit elektronenziehenden Substituenten aus der Gruppe der oben angegebenen Substituenten substituiertes Phenyl oder ein definiertes substituiertes N-Heteroaryl darstellen,

und die erhalten Verbindungen der Formel I gewünschtenfalls in ihre Salze oder Säureadditionssalze überführt.

2. Verfahren gemäß Anspruch 1, dadurch gekennzeichnet, daß Verbindungen hergestellt werden, in denen $R^1$ 1 bis 7 C-Atome enthält.

3. Verfahren gemäß Anspruch 1 oder 2, dadurch gekennzeichnet, daß Verbindungen hergestellt werden, in denen $R^1$ unverzweigtes Alkyl oder Alkenyl bedeutet.

4. Verfahren gemäß Anspruch 1 oder 2, dadurch gekennzeichnet, daß Verbindungen hergestellt werden, in denen $R^1$ verzweigtes Alkyl oder Alkenyl bedeutet.

5. Verfahren gemäß Anspruch 1 oder 2, dadurch gekennzeichnet, daß Verbindungen hergestellt werden, in denen $R^1$ einen über einen Alkylenrest mit 1 bis 6 C-Atomen mit dem N-Atom verknüpften Phenyl- oder Cycloalkylrest bedeutet.

6. Verfahren gemäß einem der vorhergehenden Ansprüche, dadurch gekennzeichnet, daß Verbindungen hergestellt werden, in denen $R^2$ und/oder $R^3$ Alkyl mit 1 bis 4 C-Atomen bedeuten.

7. Verfahren gemäß Anspruch 6, dadurch gekennzeichnet, daß Verbindungen hergestellt werden, in denen $R^2$ und/oder $R^3$ unverzweigtes Alkyl bedeuten.

8. Verfahren gemäß Anspruch 1 bis 5, dadurch gekennzeichnet, daß Verbindungen hergestellt werden, in denen $R^2$ und $R^3$ gemeinsam eine Alkylenkette $—(CH_2)_m—$ mit m = 3 bis 6 bilden.

9. Verfahren gemäß einem der vorhergehenden Ansprüche, dadurch gekennzeichnet, daß Verbindungen hergestellt werden, in denen $R^2$ und $R^3$ gleich sind.

10. Verfahren gemäß einem der vorhergehenden Ansprüche, dadurch gekennzeichnet, daß Verbindungen hergestellt werden in denen die Alkylenkette $—(CH_2)_n—$ 2 bis 6 C-Atome enthält.

11. Verfahren gemäß Anspruch 1, dadurch gekennzeichnet, daß Verbindungen hergestellt werden, in denen $R^6$ monosubstituiertes Phenyl bedeutet.

12. Verfahren gemäß Anspruch 11, dadurch gekennzeichnet, daß Verbindungen hergestellt werden, worin $R^6$ 2-Methoxy- oder 4-Chlorphenyl bedeutet.

13. Verfahren gemäß Anspruch 1, 11 oder 12, dadurch gekennzeichnet, daß Verbindungen hergestellt werden, in denen $R^1$ n-Butyl und $R^2$ und $R^3$ Methyl bedeuten.

14. Verfahren zur Herstellung von Tetraoxoverbindungen der Formel Ia und Ib

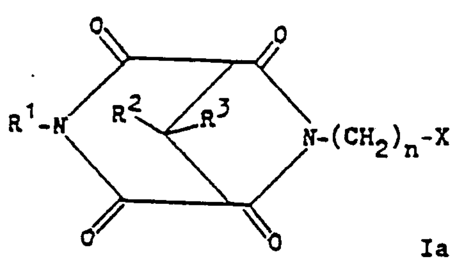

Ia

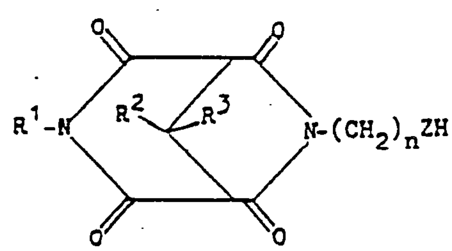

Ib

worin

$R^1$ eine Alkyl- oder Alkenylgruppe mit bis zu 12 Kohlenstoffatomen oder durch Phenyl oder Cycloalkyl mit 3—6 Kohlenstoffatomen substituiertes Alkyl mit 1—6 Kohlenstoffatomen bedeutet,

$R^2$ und $R^3$ unabhängig voneinander Alkyl mit 1—7 Kohlenstoffatomen oder Phenyl oder gemeinsam Alkylen mit 3—6 Kohlenstoffatomen bedeuten,

n 2—10 bedeutet,

X eine verdrängbare Fluchtgruppe aus der Gruppe von Halogen, Tosylat-, Mesylat-, Sulfat- und Phosphatgruppen bedeutet, und

Z die 1-Piperazinylgruppe bedeutet, dadurch gekennzeichnet, daß man

a) eine Verbindung der Formel Id

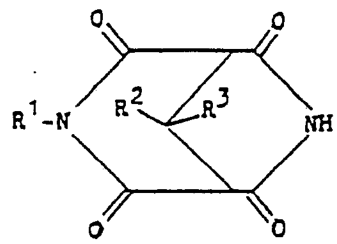

Id

worin $R^1$, $R^2$ und $R^3$ die oben angegebene Bedeutung besitzen, mit einer Verbindung der Formel (II) umsetzt

$$Y—R^5 \qquad (II)$$

worin Y die oben definierte verdrängbare Fluchtgruppe X und $R^5$ den Rest $—(CH_2)_n—X$ oder $—(CH_2)_n—ZH$, worin n, Z und X obige Bedeutung besitzen, darstellen oder

b) eine Verbindung der Formel Ia mit einer Verbindung der Formel II umsetzt, worin Y Wasserstoff und $R^5$ den ZH-Rest darstellen.

worin Y die in Anspruch 14 definierte verdrängbare Fluchtgruppe X und $R^5$ den Reste $—(CH_2)_n—X$ oder $—(CH_2)_n—ZH$, worin Z, n und X obige Bedeutung besitzen, darstellen oder

b) eine Verbindung der Formel Ia mit einer Verbindung der Formel II umsetzt, worin Y Wasserstoff und $R^5$ den ZH-Rest darstellen.

**Revendications pour les Etats contractants: BE CH DE FR GB IT LU NL SE**

1. Composés tétracarbonyle de formule (Ic)

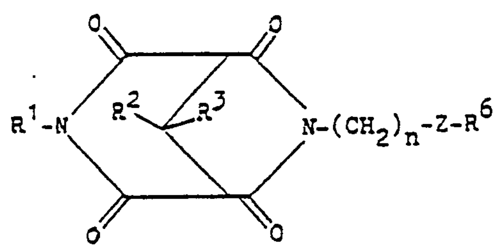

Ic

dans laquelle:

$R^1$ représente un groupe alkyle ou alcényle ayant jusqu'à 12 atomes de carbone ou un groupe alkyle ayant 1 à 6 atomes de carbone et qui est substitué par un groupe phényle ou cycloalkyle ayant 3 à 6 atomes de carbone,

$R^2$ et $R^3$ représentent, indépendamment l'un de l'autre, un groupe alkyle ayant 1 à 7 atomes de carbone ou un groupe phényle ou ils forment ensemble un groupe alkylène ayant 3 à 6 atomes de carbone,

n indique 2 à 10 atomes de carbone,

Z représente le groupe 1-pipérazinyle, et

$R^6$, situé en position 4 représente un groupe phényle non substitue' ou mono-substitué par un substituant choisi parmi le groupe alkyle ayant 1 ou 2 atomes de carbone, alcoxy ayant 1 ou 2 atomes de carbone, un atome d'halogène, un groupe hydroxy, nitro, cyano et trifluorométhyle, ou bien disubstitué par des substituants choisis parmi le groupe alkyle ayant 1 ou 2 atomes de carbone, alcoxy ayant 1 ou 2 atomes de carbone, un atome d'halogène, une groupe trifluorométhyle, méthylènedioxy et éthylènedioxy, ou un groupe N-hétéroaryle, choisi parmi le groupe 2-pyridyle, 4-pyridyle, 2-pyrimidyle et 2-pyrazinyle, éventuellement monosubstitués par un groupe alkyle ayant 1 ou 2 atomes de carbone, nitro, halogéno ou alcoxy ayant 1 ou 2 atomes de carbone,

ainsi que leurs sels et sels d'addition d'acides.

2. Composés selon la revendication 1, dans lesquels $R^1$ contient 1 à 7 atomes de carbone.

3. Composés selon la revendication 1 ou 2, dans lesquels $R^1$ représente un groupe alkyle ou alcényle non ramifié.

4. Composés selon la revendication 1 ou 2, dans lesquels $R^1$ représente un groupe alkyle ou alcényle ramifié.

5. Composés selon la revendication 1 ou 2, dans lesquels $R^1$ représente un reste phényle ou cycloalkyle, reliés à l'atome de N par l'intermédiaire d'un reste alkylène ayant 1 à 6 atomes de carbone.

6. Composés selon l'une des revendications précédentes, dans lesquels $R^2$ et/ou $R^3$ représente(nt) un groupe alkyle ayant 1 à 4 atomes de carbone.

7. Composés selon la revendication 6, dans lesquels $R^2$ et/ou $R^3$ représentent un groupe alkyle non ramifié.

8. Composés selon les revendication 1 à 5, dans lesquels $R^2$ et $R^3$ forment ensemble une chaîne alkylène $-(CH_2)_m-$, dans laquelle m vaut de 3 à 6.

9. Composés selon l'une des revendications précédentes, dans lesquels $R^2$ et $R^3$ ont la même signification.

10. Composés selon l'une des revendications précédentes, dans lesquel la chaîne alkylène $-(CH_2)_n-$ contient 2 à 6 atomes de carbone.

11. Composés selon la revendication 1, dans lesquels $R^6$ représente un groupe phényle monosubstitué.

12. Composés selon la revendication 11, dans lesquels $R^6$ représente un groupe 2-méthoxy-phényle ou 4-chorophényle.

13. Composés selon la revendication 1, 11 ou 12, dans lesquels $R^1$ représente un groupe n-butyle et $R^2$ et $R^3$ représentent chacun un groupe méthyle.

14. Composés tétracarbonyle de formule Ia et Ib

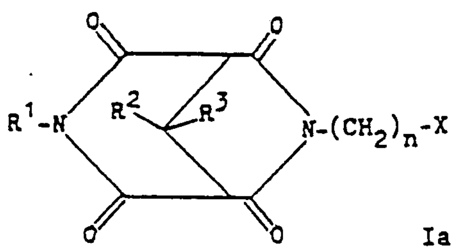

Ia

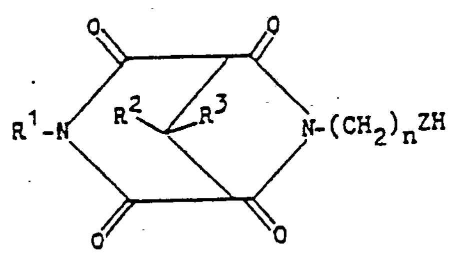

Ib

dans lesquelles

$R^1$ représente un groupe alkyle ou alcényle ayant jusqu'à 12 atomes de carbone ou bien un groupe alkyle ayant 1 à 6 atomes de carbone et substitué par un groupe phényle ou cycloalkyle ayant 3 à 6 atomes de carbone, $R^2$ et $R^3$ représentent, indépendamment l'un de l'autre, un groupe alkyle ayant 1 à 7 atomes de carbone ou un groupe phényle, ou bien ils forment ensemble un groupe alkylène ayant 3 à 6 atomes de carbone,

n vaut 2 à 10,

X représente un groupe déplaçable, choisi parmi un atome d'halogène, un groupe tosylate, mésylate, sulfate et phosphate, et

Z représente le groupe 1-pipérazinyle.

15. Médicament contenant au moins un composé selon la revendication 1 ou ses sels d'addition d'acide, pharmaceutiquement applicables.

16. Procédé pour préparer des composés de formule Ic selon la revendication 1

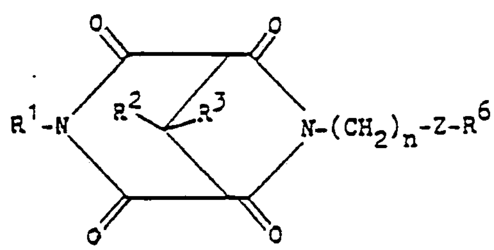

Ic

(dans lesquels $R^1$, $R^2$, $R^3$, $R^4$, n et Z ont le sens indiqué à la revendication 1) et leurs sels et sels d'addition d'acides, procédé caractérisé en ce qu'on fait réagir des composés répondant aux formules Ia ou Ib ou Id

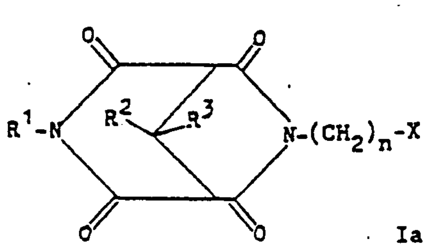

Ia

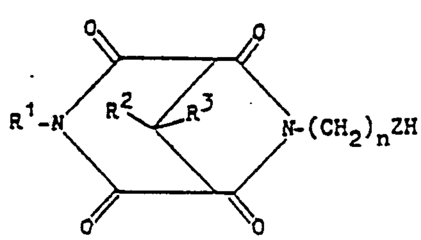

Ib

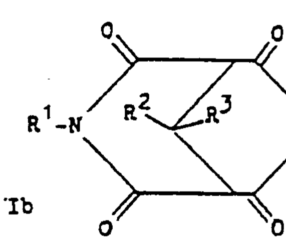

Id

(dans lesquelles $R^1$, $R^2$, $R^3$, n et Z ont la signification ci-dessus, et X a le sens indiqué à la revendication 14) avec un composé de formule générale (II):

$$Y{-}R^5 \qquad (II)$$

et

a) en cas de réaction avec un composé de formule Id,

a1) Y représente le groupe X, déplaçable défini à la revendication 14, représente un groupe $(CH_2)_n{-}Z{-}R^6$, où n, Z et $R^6$ ont le sens indiqué à la revendication 1, ou

a2) au cas où n vaut 4, Y représente le groupe

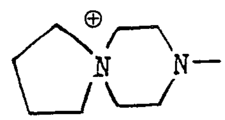

et $R^5$ représente le reste $R^6$, ou bien

b) en cas de réaction avec un composé de formule Ia,

Y représente un atome d'hydrogène et $R^5$ représente un groupe $Z{-}R^6$, où Z et $R^6$ ont le sens indiqué à la revendication 1, ou bien

c) en cas de réaction avec un composé de formule Ib

Y représente un groupe déplaçable nucléophile V, et $R^3$ représente un groupe phényle (substitué en position ortho et/ou para par des substituants attirant des électrons et choisis parmi les substituants indiqués à la revendication 1) ou un groupe N-hétéroaryle substitué selon la revendication 1

et, eventuellement, on transforme les composés de formule I, ainsi obtenus, en leurs sels ou sels d'addition d'acide.

17. Procédé pour préparer des composés de formules Ia et Ib selon la revendication 1

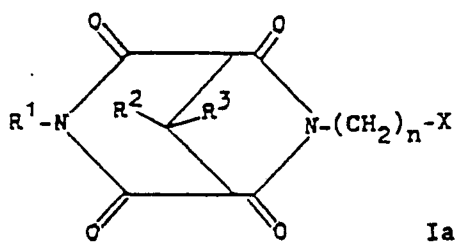

Ia

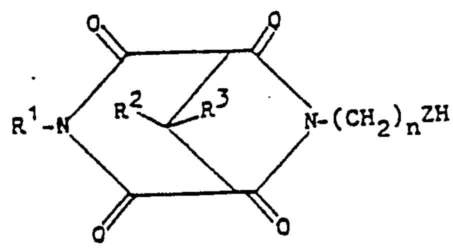

Ib

(dans lesquelles $R^1$, $R^2$, $R^3$, n, Z et X ont le sens indiqué dans la revendication 14), procédé caractérisé en ce qu'on fait réagir

a) un composé de formule Id

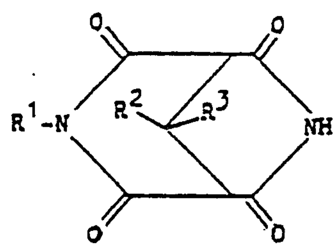

Id

(dans laquelle $R^1$, $R^2$ et $R^3$ ont le sens indiqué à la revendication 14) avec un composé de formule (II):

$$Y{-}R^5 \qquad (II)$$

dans laquelle Y représente le groupe déplaçable défini à la revendication 14, et $R^5$ représente le reste $-(CH_2)_n{-}X$ ou $-(CH_2)_n{-}ZH$, où Z, n et X ont le sens précité, ou bien

b) un composé de formule Ia avec un composé de formule II, dans lequel Y représente un atome d'hydrogène et $R^5$ représente le reste ZH.

**Revendications pour l'Etat contractant: AT**

1. Procédé pour préparer des composés tétracarbonyle de formule (Ic)

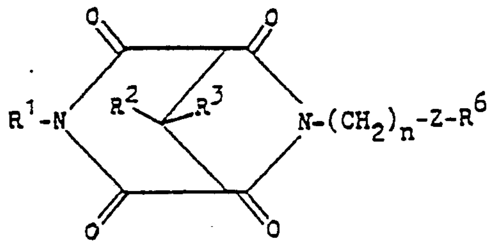

Ic

dans laquelle

$R^1$ représente un groupe alkyle ou alkylène ayant jusqu'à 12 atomes de carbone ou un groupe alkyle, ayant 1 à 6 atomes de carbone et qui et substitué par un groupe phényle ou cycloalkyle ayant 3 à 6 atomes de carbone,

$R^2$ et $R^3$ représentent, indépendamment l'un de l'autre, un groupe alkyle ayant 1 à 7 atomes de carbone ou un groupe phényle ou ils forment ensemble un groupe alkylène ayant 3 à 6 atomes de carbone,

n indique 2 à 10 atomes de carbone,

Z représente le groupe 1-pipérazinyle, et

$R^4$, présent en position 4, représente un groupe phényle non substitué ou un groupe phényle monosubstitué par un substituant choisi parmi le groupe alkyle ayant 1 ou 2 atomes de carbone, alcoxy ayant 1 ou 2 atomes de carbone, un atome d'halogène, un groupe hydroxy, nitro, cyano et trifluorométhyle, ou disubstitué par des substituants choisis parmi le groupe alkyle ayant 1 ou 2 atomes de carbone, alcoxy ayant 1 ou 2 atomes de carbone, un atome d'halogène, une groupe trifluorométhyle, méthylènedioxy et éthylènedioxy, ou un groupe N-hétéroaryle, choisi parmi le groupe 2-pyridyle, 4-pyridyle, 2-pyrimidyle et 2-pyrazinyle et éventuellement monosubstitué par un groupe alkyle ayant 1 ou 2 atomes de carbone, nitro, halogéno ou alcoxy ayant 1 ou 2 atomes de carbone,

ainsi que leurs sels et sels d'addition d'acides,

procédé caractérisé en ce qu'on fait réagir des composés de formule Ia ou Ib ou Id

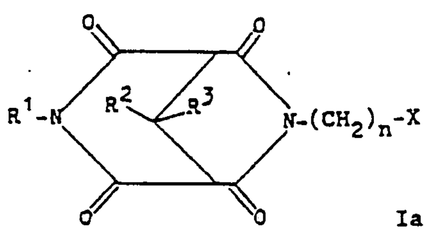

Ia

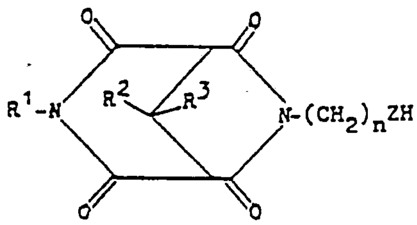

Ib

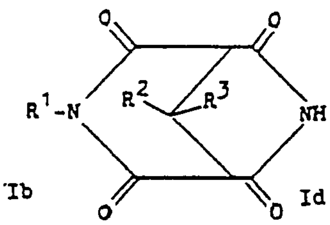

Id

dans lesquelles $R^1$, $R^2$, $R^3$, n et Z ont le sens ci-dessus, et X représente un groupe deplaçable choisi parmi des groupes halogèno, tosylate, mésylate, sulfate et phosphate) avec une composé de formule généralé (II)

$$Y—R^5 \quad (II)$$

et

a) en cas de réaction avec un composé de formule Id,

a1) Y représente le groupe déplaçable X défini ci-dessus et $R^5$ représente un groupe $(CH_2)_n—Z—R^6$, dans laquelle n, Z et $R^6$ ont le sens indiqué di-dessus, ou bien

a2) au cas où n vaut 4, Y représente le groupe

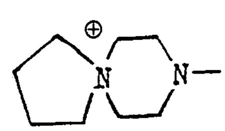

et $R^5$ représente le reste $R^6$, ou bien

b) en cas de réaction avec un composé de formule Ia, Y représente un atome d'hydrogène et $R^5$ représente un groupe $Z—R^6$, dans lequel Z et $R^6$ ont le sens indiqué ci-dessus, ou bien

c) en cas de réaction avec un composé de formule Ib

Y représente un groupe déplaçable nucléophile V et $R^5$ représente un groupe phényle (substitué en position ortho et/ou para par des substituant attirant des électrons et choisis parmi les substituants précités) ou un N-hétéroaryle substitué, défini ci-dessus, et, eventuellement, on transforme les composés de formule I ainsi obtenus en leurs sels ou sels d'addition d'acides.

2. Procédés selon la revendication 1, caractérisé en ce qu'on produit des composés, dans lequel $R^1$ contient 1 à 7 atomes de carbone.

3. Procédés selon la revendication 1 ou 2, caractérisé en ce qu'on produit des composés dans lesquels $R^1$ représente un groupe alkyle ou alcényle non ramifié.

4. Procédés selon la revendication 1 ou 2, caractérisé en ce qu'on produit des composés dans lesquels $R^1$ représente un groupe alkyle ou alcényle ramifié.

5. Procédés selon la revendication 1 ou 2, caractérisé en ce qu'on produit des composés dans lesquels $R^1$ représente un reste phényle ou cycloalkylle relié à l'atome de N par l'intermédiaire d'un reste alkylène ayant 1 à 6 atomes de carbone.

6. Procédé selon l'une des revendications précédentes, caractérisé en ce qu'on des composés, dans lesquels $R^2$ et/ou $R^3$ représente(nt) un groupe alkyle ayant 1 à 4 atomes de carbone.

7. Procédé selon la revendication 6, caractérisé en ce qu'on produit des composés dans lesquels $R^2$ et/ou $R^3$ représente(nt) un groupe alkyle non ramifié.

8. Procédé selon la revendication 1 à 5, caractérisé en ce qu'on produit des composés, dans lesquels $R^2$ et $R^3$ forment ensemble un chaîne alkylène $—(CH_2)_m—$ dans laquelle m vaut 3 à 6.

9. Procédé selon l'une des revendications précédentes, caractérisé en ce qu'on produit des composés dans lesquels $R^2$ et $R^3$ ont le même sens.

10. Procédé selon l'une des revendications précédentes, caractérisé en ce qu'on produit des composés dans lesquels la chaîne alkylène $—(CH_2)_n—$ contient 2 à 6 atomes de carbone.

11. Procédé selon la revendication 1, caractérisé en ce qu'on produit des composés dans lesquels $R^6$ représente un groupe phényle mono-substitué.

12. Procédé selon la revendication 11, caractérisé en ce qu'on produit des composés dans lesquels $R^6$ représente un groupe 2-méthoxy ou 4-chlorophényle.

13. Procédé selon la revendication 1, 11 ou 12, caractérisé en ce qu'on produit des composés dans lesquels $R^1$ représente un groupe n-butyle et $R^2$ et $R^3$ représentent chacun un groupe méthyle.

14. Procédé pour préparer des composés tétracarbonyle de formule Ia et Ib

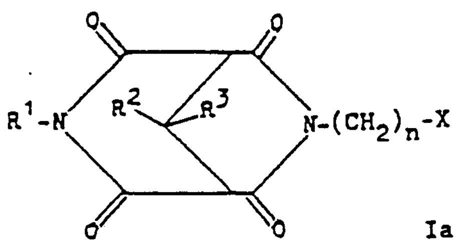
Ia

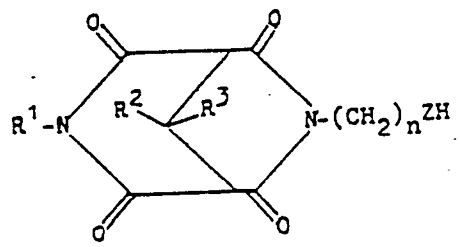
Ib

dans lesquelles:

$R^1$ représente un groupe alkyle ou alcényle ayant jusqu'à 12 atomes de carbone ou un groupe alkyle, ayant 1 à 6 atomes de carbone, substitué par un groupe phényle ou cycloalkyle ayant 3 à 6 atomes de carbone,

$R^2$ et $R^3$ représentent, indépendamment l'un de l'autre, un groupe alkyle ayant 1 à 7 atomes de carbone ou un groupe phényle, ou bien ils forment ensemble un groupe alkylène ayant 3 à 6 atomes de carbone,

n vaut 2 à 10,

X représente un groupe déplaçable choisi parmi un halogène, un groupe tosylate, mésylate, sulfate et phosphate, et

Z représente le groupe 1-pipérazinyle, procédé caractérisé en ce qu'on fait réagir:

a) un composé de formule Id

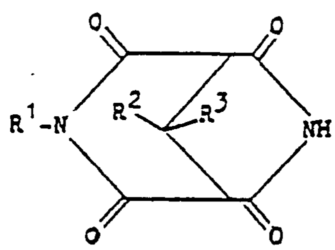
Id

(dans laquelle $R^1$, $R^2$ et $R^3$ ont le sens précité) avec un composé de formule (II)

$$Y—R^5 \qquad (II)$$

dans laquelle Y représente le groupe déplaçable X défini ci-dessus, et $R^5$ présente le reste $—(CH_2)_n—X$ ou $—(CH_2)_n$, dans lesquels n, Z et X ont le sens ci-dessus, ou

b) un composé de formule Ia avec un composé de formule II, où Y représente un atome d'hydrogène et $R^5$ représente le reste ZH.

**Claims for the Contracting States: BE CH DE FR GB IT LU NL SE**

1. Tetraoxo compounds of the Formula (Ic),

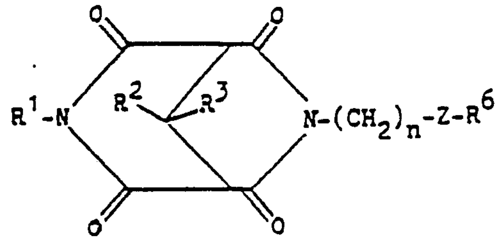

Ic

in which

$R^1$ is an alkyl or alkenyl group with up to 12 carbon atoms or alkyl with 1—6 carbon atoms substituted by phenyl or by cycloalkyl with 3—6 carbon atoms,

$R^2$ and $R^3$, independent of each other, are alkyl with 1—7 carbon atoms or phenyl or together represent alkylene with 3—6 carbon atoms,

n is 2—10 carbon atoms,

Z is the 1-piperazinyl group, and

$R^6$ is in the 4-position and is phenyl, phenyl monosubstituted by a substituent from the group alkyl with 1—2 carbon atoms, alkoxy with 1—2 carbon atoms, halogen, hydroxy, nitro, cyano and trifluoromethyl or disubstituted by substituents from the group alkyl with 1—2 carbon atoms, alkoxy with 1—2 carbon atoms, halogen, trifluoromethyl, methylenedioxy and ethylenedioxy; N-heteroaryl from the group 2-pyridyl, 4-pyridyl, 2-pyrimidyl and 2-pyrazinyl, optionally monosubstituted by alkyl with 1—2 carbon atoms, nitro, halogen or alkoxy with 1—2 carbon atoms,

and their salts and acid addition salts.

2. Compounds according to Claim 1, in which $R^1$ contains 1 to 7 C atoms.

3. Compounds according to Claim 1 or 2, in which $R^1$ is unbranched alkyl or alkenyl.

4. Compounds according to Claim 1 or 2, in which $R^1$ is branched alkyl or alkenyl.

5. Compounds according to Claim 1 or 2, in which $R^1$ is a phenyl or cycloalkyl radical linked with the N atom via an alkylene radical with 1 to 6 C atoms.

6. Compounds according to one of the preceding Claims, in which $R^2$ and/or $R^3$ are alkyl with 1 to 4 C atoms.

7. Compounds according to Claim 6, in which $R^2$ and/or $R^3$ are unbranched alkyl.

8. Compounds according to Claims 1 to 5, in which $R^2$ and $R^3$ together form an alkylene chain $—(CH_2)_m—$ with m = 3 to 6.

9. Compounds according to one of the preceding Claims, in which $R^2$ and $R^3$ are identical.

10. Compounds according to one of the preceding Claims in which the alkylene chain $—(CH_2)_n—$ contains 2 to 6 C atoms.

11. Compounds according to Claim 1, in which $R^6$ is monosubstituted phenyl.

12. Compounds according to Claim 11, in which $R^6$ is 2-methoxyphenyl or 4-chlorophenyl.

13. Compounds according to Claim 1, 11 or 12, in which $R^1$ is n-butyl and $R^2$ and $R^3$ are methyl.

14. Tetraoxo compounds of Formula Ia and Ib,

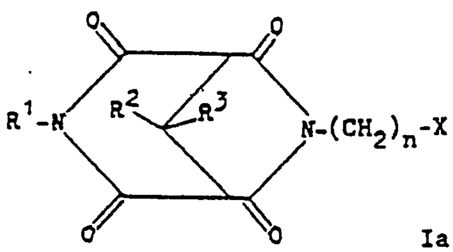

Ia

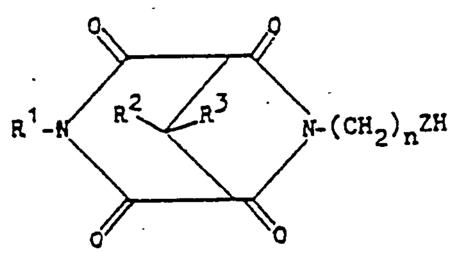

Ib

in which

$R^1$ is an alkyl or alkenyl group with up to 12 carbon atoms or alkyl with 1—6 carbon atoms substituted by phenyl or by cycloalkyl with 3—6 carbon atoms,

$R^2$ and $R^3$, independent of each other, are alkyl with 1—7 carbon atoms or phenyl or together represent alkylene with 3—6 carbon atoms,

n is 2—10,

X is a displaceable leaving group from the group of halogen, or tosylate, mesylate, sulphate and phosphate groups, and

Z is the 1-piperazinyl group.

15. Medicament containing at least one compound according to Claim 1 or the pharmaceutically usable acid addition salts thereof.

16. Method for the preparation of compounds of Formula Ic in accordance with Claim 1,

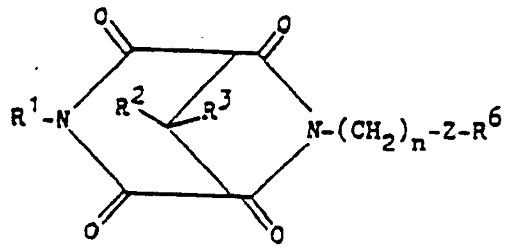

Ic

in which $R^1$, $R^2$, $R^3$, $R^6$, n and Z have the meanings given in Claim 1, and their salts and acid addition salts, characterised in that compounds of Formula Ia or Ib or Id,

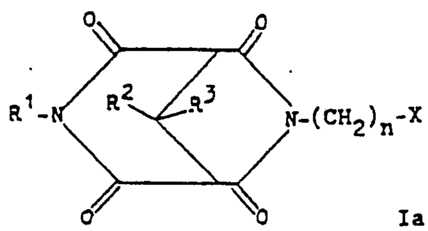

Ia

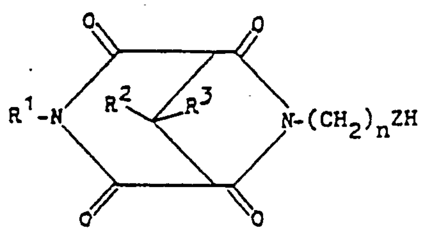

Ib

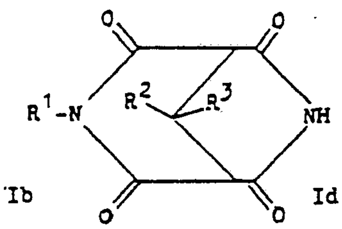

Id

in which $R^1$, $R^2$, $R^3$, n and Z have the above meanings, and X has the meaning given in Claim 14, are reacted with a compound of the general formula (II),

$$Y—R^5 \qquad (II)$$

whereby

a) in the case of reaction with a compound of Formula Id,

a1) Y represents the displaceable leaving group X defined in Claim 14 and $R^5$ represents a $(CH_2)_n—Z—R^6$ group, in which n, Z and $R^6$ have the meanings given in Claim 1, or

a2) in the case of n being 4, Y represents the group

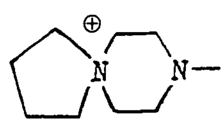

and $R^5$ represents the radical $R^6$, or

b) in the case of reaction with a compound of Formula Ia,

Y is hydrogen and $R^5$ is a $Z—R^6$ group, in which Z and $R^6$ have the meanings given in Claim 1, or

c) in the case of reaction with a compound of Formula Ib,

Y is a nucleophilically displaceable leaving group V and $R^5$ is a phenyl substituted in the ortho and/or para position with electron-drawing substituents from the group of the substituents given in Claim 1 or a substituted N-heteroaryl according to Claim 1,

and the resulting compounds of Formula I are converted if desired into their salts or acid addition salts.

17. Method for the preparation of compounds of Formula Ia and Ib according to Claim 14,

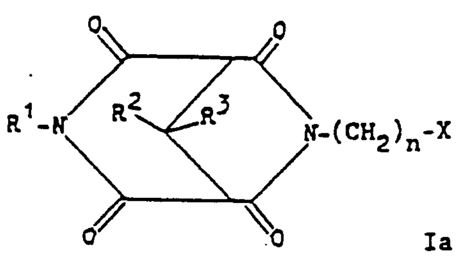

Ia

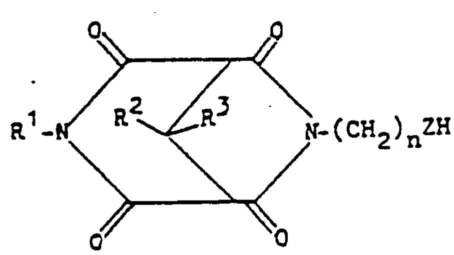

Ib

in which $R^1$, $R^2$, $R^3$, n, Z and X have the meanings given in Claim 14, characterised in that

a) a compound of Formula Id,

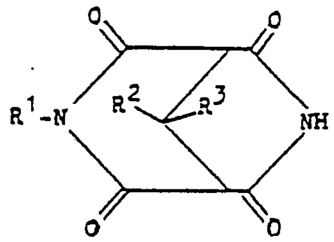

Id

in which $R^1$, $R^2$ and $R^3$ have the meanings given in Claim 14,
is reacted with a compound of Formula (II),

$$Y—R^5 \qquad (II)$$

in which Y is the displaceable leaving group X defined in Claim 14 and $R^5$ is the radical $—(CH_2)_n—X$ or $—(CH_2)_n—ZH$, in which n, Z and X have the above meanings, or

b) a compound of Formula Ia is reacted with a compound of Formula II, in which Y is hydrogen and $R^5$ is the ZH radical.

**Claims for the Contracting State: AT**

1. Method for preparing tetraoxo compounds of the Formula (Ic),

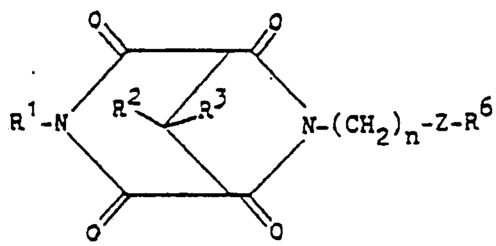

Ic

in which

$R^1$ is an alkyl or alkenyl group with up to 12 carbon atoms or alkyl with 1—6 carbon atoms substituted by phenyl or by cycloalkyl with 3—6 carbon atoms,

$R^2$ and $R^3$, independent of each other, are alkyl with 1—7 carbon atoms or phenyl or together represent alkylene with 3—6 carbon atoms,

n is 2—10 carbon atoms,

Z is the 1-piperazinyl group, and

$R^6$ is in the 4-position and is phenyl, phenyl monosubstituted by a substituent from the group alkyl with 1—2 carbon atoms, alkoxy with 1—2 carbon atoms, halogen, hydroxy, nitro, cyano and trifluoromethyl or disubstituted by substituents from the group alkyl with 1—2 carbon atoms, alkoxy with 1—2 carbon atoms, halogen, trifluoromethyl, methylenedioxy and ethylenedioxy; N-heteroaryl from the group 2-pyridyl, 4-pyridyl, 2-pyrimidyl and 2-pyrazinyl, optionally monosubstituted by alkyl with 1—2 carbon atoms, nitro, halogen or alkoxy with 1—2 carbon atoms,

and their salts and acid addition salts,

characterised in that compounds of Formula Ia or Ib or Id,

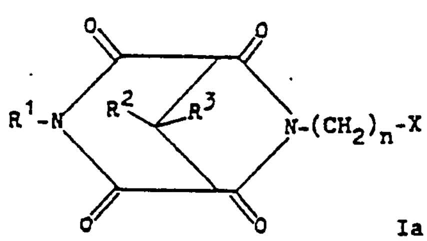

Ia

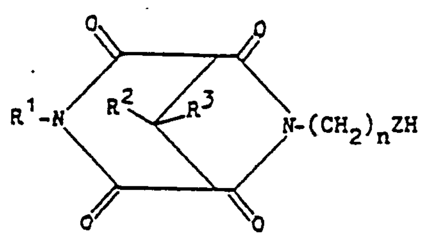

Ib

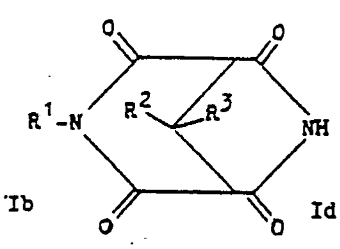

Id

in which $R^1$, $R^2$, $R^3$, n and Z have the above meanings, and X is a displaceable leaving group from the group of halogen, or tosylate, mesylate, suphate and phosphate groups, are reacted with a compound of the general formula (II),

$$Y—R^5 \qquad (II)$$

whereby

a) in the case of reaction with a compound of Formula Id,

a1) Y represents the displaceable leaving group X defined above and $R^5$ represents a $(CH_2)_n—Z—R^6$ group, in which n, Z and $R^6$ have the above meanings, or

a2) in the case of n being 4, Y represents the group

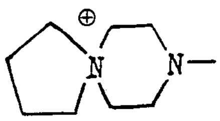

and $R^5$ represents the radical $R^6$, or

b) in the case of reaction with a compound of Formula Ia,

Y is hydrogen and $R^5$ is a $Z—R^6$ group, in which Z and $R^6$ have the above meanings,

c) in the case of reaction with a compound of Formula Ib,

Y is a nucleophilically displaceable leaving group V and $R^5$ is a phenyl substituted in the ortho and/or para position with electron-drawing substituents from the group of the substituents given above or a substituted N-heteroaryl defined above,

and the resulting compounds of Formula I are converted if desired into their salts or acid addition salts.

2. Method according to Claim 1, characterised in that compounds are produced in which $R^1$ contains 1 to 7 C atoms.

3. Method according to Claim 1 or 2, characterised in that compounds are produced in which $R^1$ is an unbranched alkyl or alkenyl.

4. Method according to Claim 1 or 2, characterised in that compounds are produced in which $R^1$ is branched alkyl or alkenyl.

5. Method according to Claim 1 or 2, characterised in that compounds are produced in which $R^1$ is a phenyl or cycloalkyl radical linked with the N atom via an alkylene radical with 1 to 6 C atoms.

6. Method according to one of the preceding Claims, characterised in that compounds are produced in which $R^2$ and/or $R^3$ are alkyl with 1 to 4 C atoms.

7. Method according to Claim 6, characterised in that compounds are produced in which $R^2$ and/or $R^3$ are unbranched alkyl.

8. Method according to Claim 1 to 5, characterised in that compounds are produced in which $R^2$ and $R^3$ together form an alkylene chain $—(CH_2)_m—$ with m = 3 to 6.

9. Method according to one of the preceding Claims, characterised in that compounds are produced in which $R^2$ and $R^3$ are identical.

10. Method according to one of the preceding Claims, characterised in that compounds are produced in which the alkylene chain $—(CH_2)_n—$ contains 2 to 6 C atoms.

11. Method according to Claim 1, characterised in that compounds are produced in which $R^6$ is monosubstituted phenyl.

12. Method according to Claim 11, characterised in that compounds are produced in which $R^6$ is 2-methoxyphenyl or 4-chlorophenyl.

13. Method according to Claim 1, 11 or 12, characterised in that compounds are produced in which $R^1$ is n-butyl and $R^2$ and $R^3$ are methyl.

14. Method for the preparation of tetraoxo compounds of Formula Ia and Ib,

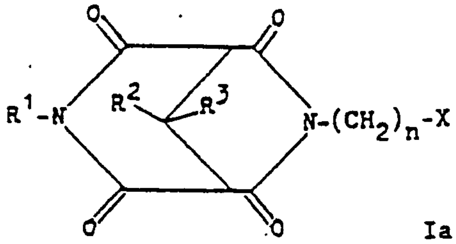

Ia

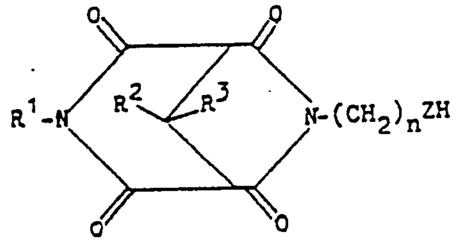

Ib

in which

$R^1$ is an alkyl or alkenyl group with up to 12 carbon atoms or alkyl with 1—6 carbon atoms substituted by phenyl or by cycloalkyl with 3—6 carbon atoms,

$R^2$ and $R^3$, independent of each other, are alkyl with 1—7 carbon atoms or phenyl or together represent alkylene with 3—6 carbon atoms,

n is 2—10,

X is a displaceable leaving group from the group of halogen, tosylate, mesylate, sulphate and phosphate groups, and

Z is the 1-piperazinyl group, characterized in that

a) a compound of Formula Id,

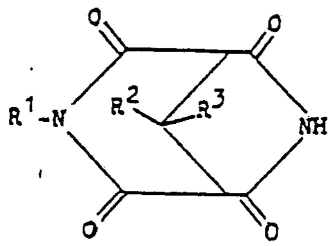

Id

in which $R^1$, $R^2$ and $R^3$ have the above meanings, is reacted with a compound of formula (II),

$$Y—R^5 \qquad (II)$$

in which Y is the displaceable leaving group X defined above and $R^5$ is the radical $—(CH_2)_n—X$ or $—(CH_2)_n—ZH$, in which n, Z and X have the above meanings, or

b) a compound of Formula Ia is reacted with a compound of Formula II, in which Y is hydrogen and $R^5$ is the ZH radical.